# EUROPEAN PATENT APPLICATION

(11) **EP 4 471 034 A1**
(43) Date of publication of application: **04.12.2024**
(21) Application number: 23893762.7
(22) Date of filing: 20.11.2023
(51) Int. Cl.: C07D 487/04, A61K 31/495, A61K 31/53, A61K 31/416, A61P 25/00, A61P 25/22, A61P 25/24

(54) **DISUBSTITUTED OCTAHYDROPYRROLO[3,4-C]PYRROLE METHYL KETONE DERIVATIVE AND APPLICATION THEREOF**

(30) Priority: 23.11.2022 CN 202211476737
(71) Applicant: Jiangsu NHWA Pharmaceutical Co., Ltd, Xuzhou, Jiangsu 221000 (CN)
(72) Inventor: DOU, Fei, Xuzhou, Jiangsu 221000 (CN); DONG, Yingying, Xuzhou, Jiangsu 221000 (CN); DOU, Pengfei, Xuzhou, Jiangsu 221000 (CN); GE, Meng, Xuzhou, Jiangsu 221000 (CN); XU, Xiangqing, Xuzhou, Jiangsu 221000 (CN)
(74) Representative: Lavoix
(86) International application number: PCT/CN2023/132527
(87) International publication number: WO 2024/109672

(57) **Abstract**

The present invention belongs to the field of medicine, and particularly relates to a compound represented by general formula I, which is shown below, or a pharmaceutically acceptable salt, a stereoisomer, or a tautomer, and a composition comprising the compound, a preparation method therefor, and use thereof in the field of medicine.

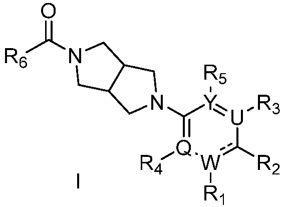

## Description

### TECHNICAL FIELD

The present invention belongs to the field of medicine, and particularly relates to a disubstituted octahydropyrrolo[3,4-*c*]pyrrolyl methyl ketone derivative or a pharmaceutically acceptable salt thereof, a stereoisomer thereof, or a tautomer thereof, and a composition including the compound, and use thereof in the field of medicine.

### BACKGROUND

Orexin (also known as hypocretin or orexigenic peptides) exists in two types: orexin-A (hypocretin-1) and orexin-B (hypocretin-2). Orexin signaling is mediated by two receptors and two peptide agonists, where orexin A and orexin B bind to two high affinity receptors orexin receptor type 1 (OX1R or OX1) and orexin receptor type 2 (OX2R or OX2). OX1R tends to bind to orexin A, while OX2R binds both orexins with similar affinity.

Several lines of evidence suggest: the wakefulness-promoting effects mediated by orexin are associated with the projection of orexin neurons to histaminergic neurons in the tuberomammillary nucleus (Yamanaka et al., 2002, Biochem. Biophys. Res. Comm., 290: 1237-1245). Clinical evidence further supports that orexin signaling is a target for sleep-promoting therapies, as observed by reduced orexin levels and loss of orexin-producing neurons in human narcolepsy patients (Mignot et al., 2001, The American Journal of Human Genetics, 68: 686-699) or, in rare cases, is associated with mutations in the OX2R receptor gene (Peyron et al., 2000, Naturemed., 6: 991-997).

Thus, orexin receptors are pathologically significant and are associated with a variety of diseases, such as sleep disorder, depression, anxiety disorder, panic disorder, obsessive-compulsive disorder, affective neuropathy, depressive neuropathy, anxiety neuropathy, mood disorder, panic attack disorder, behavioral disorder, emotional disturbance, post-traumatic stress disorder, psychosis, schizophrenia, manic depression, delirium, dementia, drug dependence, addiction, cognitive disorder, Alzheimer's disease, Parkinson's disease, dyskinesia, eating disorder, headache, migraine, pain, and the like.

Research shows that: the disorder of the sleep-wake cycle is very likely to be a target of the activity of OX2R receptor modulators. Examples of disorders that are treatable with antagonists or other modulators that down-regulate OX2R-mediated processes include insomnia, restless legs syndrome, jet lag (difficulty in sleeping), and sleep disorders secondary to neurological disorders such as mania, schizophrenia, pain syndromes, and the like. OX2R is selectively expressed in the tuberomammillary nucleus (TMN), hypothalamic paraventricular nucleus (PVN), and nucleus accumbens (NAc), which are major effector sites of orexin neurons in the lateral hypothalamus LH and are associated with feeding, sleep, depression, anxiety, drug addiction, and motivational behaviors, showing greater efficacy in the treatment of sleep disorders (Lu et al., 2020, Neurosci Bull, 4: 432-448).

### SUMMARY

The present invention provides a class of compounds with orexin receptor antagonistic activity. The compounds of the present invention have relatively good selectivity and pharmacodynamic activity, as well as excellent physicochemical properties and pharmacokinetic properties, and therefore have relatively good clinical application prospects.

The following is merely a summary of some aspects of the present invention, and the present invention is not limited thereto. When the disclosure of this specification differs from the cited documents, the disclosure of the present specification shall prevail.

The present invention aims to provide a disubstituted octahydropyrrolo[3,4-c]pyrrolyl methyl ketone derivative or a pharmaceutically acceptable salt, a stereoisomer, or a tautomer, and a pharmaceutical composition thereof, and the compound and the pharmaceutical composition can be used for preventing or treating an orexin receptor-related disease.

In one aspect, the present invention provides a compound represented by general formula I, which is shown below, or a pharmaceutically acceptable salt, a stereoisomer, or a tautomer, 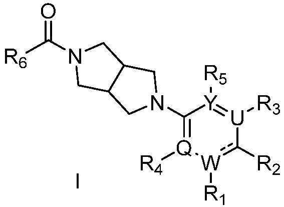
wherein R₁, R₂, R₃, R₄, and R₅ are absent or independently selected from H, halogen, C₁-C₈ linear or branched alkyl, and C₁-C₈ alkoxy;
or R₁ and R₂ form C₃-C₈ cycloalkyl, aryl, heteroaryl, or a 3-8 membered heterocyclic ring;
Q, W, Y, and U are independently selected from C and N, and Y and Q are not simultaneously C;
--- is a single bond or a double bond;
R₆ is selected from formula II, formula III, and formula IV: 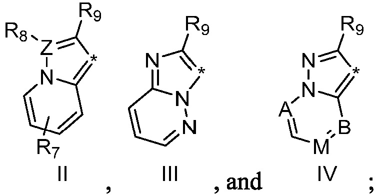
R₉ is optionally substituted heteroaryl, an optionally substituted aromatic ring, an optionally substituted 3-8 membered heterocyclic ring, or optionally substituted C₃-C₈ cycloalkyl, and a substituent is selected from halogen, C₁-C₈ linear or branched alkyl, C₁-C₈ alkoxy, and haloalkyl;
in formula II, Z is selected from C and N;
R₇ is selected from H, halogen, and C₁-C₈ linear or branched alkyl;
R₈ is absent or independently selected from H, halogen, and C₁-C₈ linear or branched alkyl;
in formula IV, A, B, and M are selected from CH and N, and A, B, and M are not simultaneously CH.

It should be understood that when --- is a single bond, W may actually also be CH. In another aspect, the present invention provides a pharmaceutical composition comprising a therapeutically effective amount of the compound represented by formula I or the pharmaceutically acceptable salt, the stereoisomer, or the tautomer described above, and optionally further comprising a pharmaceutically acceptable excipient, a carrier, an adjuvant, a vehicle, or a combination thereof.

In another aspect, the present invention provides use of a compound represented by formula I or a pharmaceutically acceptable salt, a stereoisomer, or a tautomer, and a pharmaceutical composition thereof in the preparation of a medicament for treating an orexin receptor-related disease.

In one embodiment, the orexin-related disease is sleep disorder, depression, anxiety disorder, panic disorder, obsessive-compulsive disorder, affective neuropathy, depressive neuropathy, anxiety neuropathy, mood disorder, panic attack disorder, behavioral disorder, emotional disturbance, post-traumatic stress disorder, psychosis, schizophrenia, manic depression, delirium, dementia, drug dependence, addiction, cognitive disorder, Alzheimer's disease, Parkinson's disease, dyskinesia, eating disorder, headache, migraine, pain, or the like.

In another embodiment, the orexin-related disease is sleep disorder.

### DETAILED DESCRIPTION

Unless otherwise specified or there is an obvious conflict in the context, all technical and scientific terms used herein have the same meanings as commonly understood by one of ordinary skill in the art to which the present invention belongs. In the event of a contradiction, the definition provided in the present application shall prevail. When a trade name appears herein, it is intended to refer to its corresponding commercial product or its active ingredient. All patents, published patent applications, and publications cited herein are incorporated herein by reference.

### General Terms and Definitions

The term "optional" or "optionally" means that a subsequently described event or situation may, but does not necessarily, occur, and that the description includes situations in which the event or situation occurs as well as situations in which it does not occur.

The term "optionally substituted" is used interchangeably with the term "substituted or unsubstituted", i.e., the structure or group is unsubstituted or substituted with one or more of the substituents described in the present invention, wherein the substitution occurs at any reasonable position on the given structure or group as permitted by valence.

Unless otherwise indicated, as used herein, the point of attachment of a substituent may be from any suitable position of the substituent. When a bond of a substituent is shown to pass through a bond connecting two atoms in a ring, the substituent may be bonded to any one of the ring-forming atoms in the substitutable ring.

In general, the term "substituted" means that one or more hydrogen atoms in a given structure or group are substituted with a particular substituent. Unless otherwise indicated, substitution with one substituent may occur at each reasonable substitutable position of a group. When more than one position in a given structural formula can be substituted with one or more specific substituents selected, substitution with the substituents may occur identically or differently at each reasonable position in the structural formula.

In addition, it should be noted that unless otherwise explicitly indicated, the description "each independently" used in the present invention should be understood in a broad sense, and it may mean that specific items expressed by the same symbol in different groups do not affect each other, or that specific items expressed by the same symbol in the same group do not affect each other.

When the lower and upper limits of a range of numerical values are disclosed, any numerical value falling within the range and any included range are specifically disclosed. In particular, each range of values disclosed herein is to be understood as indicating each numerical value and range encompassed within a relatively broad range. When any variable (e.g., R), as well as labeled variables (e.g., R₁, R₂, R₃, R₄, R₅, R₆, R₇, etc.), occurs more than once in a composition or structure of a compound, the variable is independently defined in each case at each occurrence. For example, if a group is substituted with 0, 1, 2, 3, or 4 R substituents, the group may optionally be substituted with up to four R substituents, and the options for each R substituent in each case are independent of each other.

In each part of this specification, the substituents of the compounds disclosed in the present invention are disclosed according to group types or ranges. It is specifically noted that the present invention includes each independent secondary combination of each member of these group types and ranges. For example, the expression m-n used herein refers to the range of m to n as well as to sub-ranges consisting of the point values therein and the point values.

The "*" in the present invention denotes a point of attachment. For example, in 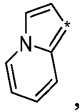 it is indicated that the substituent is attached at the "*". The term "alkyl" refers to a linear or branched saturated aliphatic hydrocarbon group consisting of carbon atoms and hydrogen atoms, which is attached to the rest of the molecule through a single bond. "Alkyl" may have 1-8 carbon atoms, i.e., "C₁-C₈ alkyl", or 1 to 6 carbon atoms, i.e., "C₁-C₆ alkyl", e.g., C₁₋₄ alkyl, C₁₋₃ alkyl, C₁₋₂ alkyl, C₃ alkyl, C₄ alkyl, C₁₋₆ alkyl, or C₃₋₆ alkyl. It may also have 1 to 3 carbon atoms, i.e., "C₁-C₃ alkyl", e.g., C₁₋₃ alkyl, C₁₋₂ alkyl, or C₃ alkyl. The term "C₁-C₅ alkyl" especially refers to independently disclosed methyl, ethyl, C₃ alkyl, C₄ alkyl, or C₅ alkyl. Examples of the alkyl group include, but are not limited to, methyl (Me, -CH₃), ethyl (Et, -CH₂CH₃), n-propyl (n-Pr, -CH₂CH₂CH₃), isopropyl (i-Pr, -CH(CH₃)₂), n-butyl (n-Bu, -CH₂CH₂CH₂CH₃), isobutyl (i-Bu, -CH₂CH(CH₃)₂), sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, and the like. For example, the expression "C₂-C₈" or "C₂-₈" encompasses a range of 2-8 carbon atoms and should be understood as also encompassing any sub-range and each point value therein, e.g., C₂-C₅, C₃-C₄, C₂-C₆, C₃-C₆, C₄-C₆, C₄-C₇, C₄-C₈, C₂-C₄, and the like, as well as C₂, C₃, C₄, C₅, C₆, C₇, C₈, and the like. As another example, the expression "C₁-C₅" or "C₁₋₅" encompasses a range of 1-5 carbon atoms and should be understood as also encompassing any sub-range and each point value therein, e.g., C₂-C₅, C₃-C₄, C₁-C₂, C₁-C₃, C₁-C₄, C₁-C₅, and the like, as well as C₁, C₂, C₃, C₄, C₅, and the like. As another example, the expression "C₂-C₅" or "C₂₋₅" encompasses a range of 2-5 carbon atoms and should be understood as also encompassing any sub-range and each point value therein, e.g., C₂-C₅, C₃-C₄, C₂-C₃, C₂-C₄, C₃-C₅, C₄-C₅, and the like, as well as C₂, C₃, C₄, C₅, and the like. As another example, the expression "C₁-C₈" or "C₁₋₈" encompasses a range of 1-8 carbon atoms and should be understood as also encompassing any sub-range and each point value therein, e.g., C₂-C₅, C₃-C₄, C₂-C₆, C₃-C₆, C₄-C₆, C₄-C₇, C₄-C₈, C₂-C₄, and the like, as well as C₁, C₂, C₃, C₄, C₅, C₆, C₇, C₈, and the like. As another example, the expression "three to eight membered" should be understood as encompassing any sub-range and each point value therein, e.g., three to five membered, three to six membered, three to seven membered, three to eight membered, four to five membered, four to six membered, four to seven membered, four to eight membered, five to seven membered, five to eight membered, six to seven membered, six to eight membered, and the like, as well as three membered, four membered, five membered, six membered, seven membered, eight membered, and the like. Other similar expressions herein should also be understood in a similar manner.

The term "one or more" or the similar expression "at least one" may indicate, for example, 1, 2, 3, 4, 5, 6, 7, 8, 9, 10 or more.

The term "haloalkyl" refers to an alkyl group substituted with one or more halogen atoms, wherein the alkyl group has the meaning described herein, and such examples include, but are not limited to, -CF₃, -CH₂F, -CHF₂, -CF₂CF₃, -CH₂CF₃, -CH₂CH₂F, CH₂CF₂CHF₂, and the like. "Haloalkyl" may have 1-8 carbon atoms, i.e., C₁₋₈ haloalkyl. In one embodiment, "haloalkyl" is a lower C₁₋₄ haloalkyl, wherein the "C₁₋₄ haloalkyl" includes fluorine-substituted C₁₋₄ alkyl, chlorine-substituted C₁₋₄ alkyl, bromine-substituted C₁₋₄ alkyl, iodine-substituted C₁₋₄ alkyl, and the like. Specifically, the fluorine-substituted C₁₋₄ alkyl includes -CH₂F, -CHF₂, -CF₃, -CH₂Cl, -CHCl₂, -CCl₃, -CH₂Br, -CHBr₂, -CBr₃, -CH₂CH₂F, -CH₂CHF₂, -CH₂CF₃, -CF₂CH₂F, -CF₂CHF₂, -CF₂CF₃, -CHFCF₃, -CHFCHF₂, -CHFCH₂F, -CH₂CH₂CF₃, -CH₂CF₂CHF₂, and the like. The haloalkyl is optionally substituted with one or more substituents described in the present invention.

The term "selected from..." refers to independent selection of one or more elements from the group listed afterward, and may include a combination of two or more elements.

The terms "comprise", "comprises", "comprising", "include", "includes", and "including" are open-ended, i.e., including what is indicated in the present invention, but not excluding other aspects.

When it is stated that each carbon atom in a group may optionally be replaced by a heteroatom, the proviso is that the normal valence of all atoms in the group in the present case is not exceeded, and that a stable compound is formed.

The term "heteroatom" means one or more oxygen (O), sulfur (S), or nitrogen (N) atoms, including any form of nitrogen (N) or sulfur (S) in an oxidation state; forms of primary, secondary, and tertiary amines and quaternary ammonium salts; or forms in which hydrogen on a nitrogen atom in a heterocyclic ring is substituted, such as: N, NH, and NR.

The term "aryl" refers to a monovalent or multivalent monocyclic, bicyclic, or tricyclic carbocyclic ring system containing 6-14 ring atoms, or 6-10 ring atoms, or 6 ring atoms, wherein at least one ring is aromatic. The aryl group is generally, but not necessarily, attached to a parent molecule through an aromatic ring of the aryl group. The term "aryl" may be used interchangeably with the term "aromatic ring". Examples of the aryl group may include phenyl, naphthyl, anthracene, and the like.

The aryl group is optionally substituted with one or more substituents described in the present invention.

The term "heteroaryl" contains 5-14 ring atoms, or 5-10 ring atoms, or 5-6 ring atoms (i.e., 5-6 membered), monovalent or multivalent monocyclic, bicyclic, or tricyclic ring systems, wherein at least one ring is aromatic and at least one ring comprises one or more heteroatoms. The heteroaryl group is generally, but not necessarily, attached to a parent molecule through an aromatic ring of the heteroaryl group. The term "heteroaryl" may be used interchangeably with the term "heteroaromatic ring" or "heteroaromatic compound". Examples of the heteroaryl ring include 5-10 membered monocyclic or bicyclic heteroaryl groups containing 1-5 heteroatoms independently selected from nitrogen, oxygen, or sulfur, including but not limited to: pyridyl, pyridazinyl, triazinyl, pyrimidinyl, thienyl, furanyl, oxazolyl, thiazolyl, thiadiazolyl, oxadiazolyl, isoxazolyl, pyrazolyl, imidazolyl, pyrrolyl, pyranyl, pyridazinyl, pyrazinyl, and triazolyl. The heteroaryl group is optionally substituted with one or more substituents described in the present invention.

The terms "heterocyclic ring" and "heterocyclyl" are used interchangeably to mean monovalent or multivalent monocyclic, bicyclic, or tricyclic ring systems comprising 3-12 ring atoms or 3-8 ring atoms, wherein one or more atoms on the ring are independently replaced by heteroatoms, the heteroatoms have the meaning described in the present invention, and the ring may be fully saturated or comprise one or more unsaturations. Unless otherwise specified, heterocyclyl may be a carbon group or a nitrogen group, and the -CH₂- group may optionally be replaced with -C(=O)-. The sulfur atom of the ring may optionally be oxidized to a S-oxide. The nitrogen atom of the ring may optionally be oxidized to a N-oxide. Examples of heterocyclyl include 3-8 membered monocyclic or bicyclic heterocyclic rings containing 1-3 heteroatoms independently selected from nitrogen, oxygen, or sulfur, including but not limited to: oxiranyl, azetidinyl, oxetanyl, thietanyl, pyrrolidinyl, 2-pyrrolinyl, 3-pyrrolinyl, pyrazolinyl, pyrazolidinyl, imidazolinyl, imidazolidinyl, tetrahydrofuranyl, dihydrofuranyl, tetrahydrothienyl, dihydrothienyl, 1,3-dioxocyclopentyl, dithiocyclopentyl, tetrahydropyranyl, dihydropyranyl, 2H-pyranyl, 4H-pyranyl, tetrahydrothiopyranyl, piperidinyl, morpholinyl, thiomorpholinyl, piperazinyl, and the like. Examples of heterocyclyl in which a -CH₂- group is replaced with -C(=O)-include, but are not limited to, 2-oxopyrrolidinyl, oxo-1,3-thiazolidinyl, 2-piperidinonyl, 3,5-dioxopiperidinyl, and pyrimidinedionyl. Examples of heterocyclyl in which the sulfur atom is oxidized include, but are not limited to, sulfolanyl and 1,1-dioxothiomorpholinyl. The heterocyclyl group may optionally be substituted with one or more substituents described in the present invention.

The term "hydrogen (H)" means a single hydrogen atom, and such an atomic group may be attached to other groups, for example, to an oxygen atom to form a hydroxyl group.

The term "halogen" or "halo" should be understood to mean fluorine (F), chlorine (Cl), bromine (Br), or iodine (I), preferably a fluorine, chlorine, or bromine atom, and more preferably a fluorine atom.

The term "alkoxy" refers to an alkyl group attached to the rest of a molecule through an oxygen atom, wherein the alkyl group has the meaning described in the present invention. Unless otherwise specified in detail, the alkoxy group contains 1 to 8 carbon atoms. In one embodiment, the alkoxy group contains 1 to 5 carbon atoms; in another embodiment, the alkoxy group contains 1 to 3 carbon atoms. The alkoxy group may optionally be substituted with one or more substituents described in the present invention. Examples of the alkoxy group include, but are not limited to, methoxy (MeO, -OCH₃), ethoxy (EtO, -OCH₂CH₃), 1-propoxy (n-PrO, n-propoxy, -OCH₂CH₂CH₃), 2-propoxy (i-PrO, i-propoxy, -OCH(CH₃)₂), 1-butoxy (n-BuO, n-butoxy, -OCH₂CH₂CH₂CH₃), 2-methyl-1-propoxy (i-BuO, i-butoxy, -OCH₂CH(CH₃)₂), 2-butoxy (s-BuO, s-butoxy, -OCH(CH₃)CH₂CH₃), 2-methyl-2-propoxy (t-BuO, t-butoxy, -OC(CH₃)₃), and the like.

The term "cycloalkyl" refers to a saturated or unsaturated cyclic hydrocarbon group consisting of carbon atoms and hydrogen atoms, which preferably comprises 1 or 2 rings. The cycloalkyl may be of a monocyclic, fused polycyclic, bridged cyclic, or spiro cyclic structure. Cycloalkyl may have 3 to 8 carbon atoms, i.e., "C₃-C₈ cycloalkyl", e.g., C₆ cycloalkyl, C₅ cycloalkyl, C₄ cycloalkyl, or C₃ cycloalkyl.

Non-limiting examples of cycloalkyl include, but are not limited to, cyclopropyl, cyclobutyl, cyclopentyl, cyclopentenyl, cyclohexyl, and the like. The term also encompasses the case where the C atom may be substituted with oxo (=O).

The term "stereoisomer" refers to a compound having the same chemical structure, but having a different arrangement of atoms or groups in space. Stereoisomers include enantiomers, diastereomers, conformational isomers (rotamers), geometric isomers (cis/trans) isomers, atropisomers, and the like.

The term "tautomer" refers to a structural isomer having different energies that are interconvertible via a low energy barrier. If tautomerism is possible (e.g., in a solution), a chemical equilibrium of tautomers can be achieved. For example, proton tautomers (also known as prototropic tautomers) include interconversion via migration of a proton, such as keto-enol isomerization and imine-enamine isomerization.

The term "pharmaceutically acceptable salt" refers to an organic salt or an inorganic salt of the compound of the present invention.

The term "pharmaceutically acceptable carrier" refers to those substances that do not have a significant irritating effect on organisms and do not impair the biological activity and properties of the active compound. The "pharmaceutically acceptable carrier" includes, but is not limited to, a glidant, a sweetener, a diluent, a preservative, a dye/colorant, a flavoring agent, a surfactant, a wetting agent, a dispersant, a disintegrant, a stabilizer, a solvent, or an emulsifier.

The following detailed description of the present invention is intended to illustrate non-limiting embodiments and to enable others skilled in the art to more fully understand the technical solutions of the present invention, its principles, and its practical applications, so that others skilled in the art may modify and implement the present invention in various forms to allow it to be optimally adapted to the requirements of particular use.

### Compound of Formula I

In one aspect, the present invention provides a compound represented by formula I or a pharmaceutically acceptable salt thereof, a stereoisomer thereof, or a tautomer thereof, 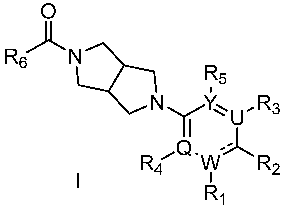
wherein R₁, R₂, R₃, R₄, and R₅ are absent or independently selected from H, halogen, C₁-C₈ linear or branched alkyl, and C₁-C₈ alkoxy;
or R₁ and R₂ form C₃-C₈ cycloalkyl, aryl, heteroaryl, or a 3-8 membered heterocyclic ring; preferably, R₁ and R₂ form C₃-C₈ cycloalkyl, 6-10 membered monocyclic or bicyclic aryl, 5-10 membered monocyclic or bicyclic heteroaryl containing 1-5 heteroatoms independently selected from nitrogen, oxygen, or sulfur, or a 3-8 membered monocyclic or bicyclic heterocyclic ring containing 1-3 heteroatoms independently selected from nitrogen, oxygen, or sulfur;
Q, W, Y, and U are independently selected from C and N, and Y and Q are not simultaneously C;
--- is a single bond or a double bond;
R₆ is selected from formula II, formula III, and formula IV: 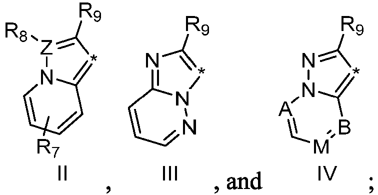
R₉ is optionally substituted heteroaryl, an optionally substituted aromatic ring, an optionally substituted 3-8 membered heterocyclic ring, and optionally substituted C₃-C₈ cycloalkyl, and a substituent is selected from halogen, C₁-C₈ linear or branched alkyl, C₁-C₈ alkoxy, and haloalkyl; preferably, R₉ is optionally substituted 5-10 membered monocyclic or bicyclic heteroaryl containing 1-5 heteroatoms independently selected from nitrogen, oxygen, or sulfur, an optionally substituted 6-10 membered monocyclic or bicyclic aromatic ring group, an optionally substituted 3-8 membered monocyclic or bicyclic heterocyclic ring containing 1-3 heteroatoms independently selected from nitrogen, oxygen, or sulfur, or optionally substituted C₃-C₈ cycloalkyl;
in formula II, Z is selected from C and N;
R₇ is selected from H, halogen, and C₁-C₈ linear or branched alkyl;
R₈ is absent or independently selected from H, halogen, and C₁-C₈ linear or branched alkyl;
in formula IV, A, B, and M are selected from CH and N, and A, B, and M are not simultaneously CH.

In one embodiment, R₁ is absent or selected from H, halogen, C₁-C₈ linear or branched alkyl, and C₁-C₈ alkoxy. In one preferred embodiment, R₁ is absent. In one preferred embodiment, R₁ is C₁-C₈ linear or branched alkyl, or C₁-C₈ alkoxy. In one more preferred embodiment, R₁ is C₁-C₅ linear or branched alkyl, or C₁-C₅ alkoxy. In one specific embodiment, R₁ is selected from methyl, ethyl, propyl, isopropyl, butyl, isobutyl, pentyl, methoxy, ethoxy, propoxy, and butoxy. In one more specific embodiment, R₁ is selected from methyl, ethyl, propyl, isopropyl, methoxy, ethoxy, and propoxy. In one particularly specific embodiment, R₁ is methyl. In another particularly specific embodiment, R₁ is ethyl. In yet another particularly specific embodiment, R₁ is methoxy. In yet another particularly specific embodiment, R₁ is ethoxy.

In one embodiment, R₂ is absent or selected from H, halogen, C₁-C₈ linear or branched alkyl, and C₁-C₈ alkoxy. In one preferred embodiment, R₂ is H and halogen. In one more preferred embodiment, R₂ is H. In one particularly preferred embodiment, R₂ is halogen. In one specific embodiment, R₂ is selected from H, fluorine, chlorine, bromine, and iodine. In one more specific embodiment, R₂ is selected from H, fluorine, chlorine, and bromine. In one particularly specific embodiment, R₂ is H. In another particularly specific embodiment, R₂ is fluorine. In yet another particularly specific embodiment, R₂ is chlorine.

In one embodiment, R₁ and R₂ form C₃-C₈ cycloalkyl, aryl, heteroaryl, or a 3-8 membered heterocyclic ring. In one preferred embodiment, R₁ and R₂ form C₃-C₆ cycloalkyl. In one embodiment, R₁ and R₂ form C₆-C₁₀ aryl. In another preferred embodiment, R₁ and R₂ form C₃-C₆ aryl. In one embodiment, R₁ and R₂ form 5-10 membered heteroaryl, preferably 5-6 membered heteroaryl. In another preferred embodiment, R₁ and R₂ form C₃-C₆ heteroaryl. In another preferred embodiment, R₁ and R₂ form a 3-6 membered heterocyclic ring. In one specific embodiment, R₁ and R₂ form cyclopropane, cyclobutane, cyclopentane, cyclopentenyl, or cyclohexane. In one specific embodiment, R₁ and R₂ form a benzene ring. In another more specific embodiment, R₁ and R₂ form cyclopentenyl. In another specific embodiment, R₁ and R₂ form pyridine. In another specific embodiment, R₁ and R₂ form thiophene. In another specific embodiment, R₁ and R₂ form pyrrole. In another specific embodiment, R₁ and R₂ form furan.

In one embodiment, R₃ is absent or selected from H, halogen, C₁-C₈ linear or branched alkyl, and C₁-C₈ alkoxy. In one preferred embodiment, R₃ is absent. In one preferred embodiment, R₃ is C₁-C₈ linear or branched alkyl, or C₁-C₈ alkoxy. In one more preferred embodiment, R₃ is C₁-C₅ linear or branched alkyl, or C₁-C₅ alkoxy. In one specific embodiment, R₃ is selected from methyl, ethyl, propyl, isopropyl, butyl, isobutyl, pentyl, methoxy, ethoxy, propoxy, and butoxy. In one more specific embodiment, R₃ is selected from methyl, ethyl, propyl, isopropyl, methoxy, ethoxy, and propoxy. In one particularly specific embodiment, R₃ is methyl. In another particularly specific embodiment, R₃ is ethyl. In yet another particularly specific embodiment, R₃ is methoxy. In yet another particularly specific embodiment, R₃ is ethoxy.

In one embodiment, R₄ and R₅ are absent or independently selected from H, halogen, C₁-C₈ linear or branched alkyl, and C₁-C₈ alkoxy.

In one embodiment, R₄ is C₁-C₈ linear or branched alkyl. In one preferred embodiment, R₄ is H. In one more preferred embodiment, R₄ is absent. In one more specific embodiment, R₄ is methyl, ethyl, or propyl. In one particularly specific embodiment, R₄ is methyl.

In one embodiment, R₅ is C₁-C₈ linear or branched alkyl. In one preferred embodiment, R₅ is H. In one more preferred embodiment, R₅ is absent. In one more specific embodiment, R₅ is methyl, ethyl, or propyl. In one particularly specific embodiment, R₅ is methyl.

In one embodiment, R₇ is selected from H, halogen, and C₁-C₈ linear or branched alkyl. In one preferred embodiment, R₇ is C₁-C₅ linear or branched alkyl, H, or halogen. In one more preferred embodiment, R₇ is H. In one particularly preferred embodiment, R₇ is halogen. In one specific embodiment, R₇ is selected from methyl, ethyl, propyl, or isopropyl. In one more specific embodiment, R₇ is selected from H. In one particularly specific embodiment, R₇ is fluorine, chlorine, bromine, or iodine. In another particularly specific embodiment, R₇ is methyl. In yet another particularly specific embodiment, R₇ is fluorine. In yet another particularly specific embodiment, R₇ is chlorine.

In one embodiment, R₈ is absent or selected from H, halogen, and C₁-C₈ linear or branched alkyl. In one preferred embodiment, R₈ is absent. In one preferred embodiment, R₈ is C₁-C₈ linear or branched alkyl. In one more preferred embodiment, R₈ is halogen. In one particularly preferred embodiment, R₈ is H. In another particularly preferred embodiment, Rg is absent. In one specific embodiment, R₈ is selected from methyl, ethyl, and propyl. In one more specific embodiment, R₈ is selected from H. In one particularly specific embodiment, R₈ is fluorine, chlorine, bromine, or iodine. In another particularly specific embodiment, R₈ is methyl. In yet another particularly specific embodiment, R₈ is fluorine. In yet another particularly specific embodiment, Rg is absent.

In one embodiment, R₉ is optionally substituted heteroaryl, an optionally substituted aromatic ring, an optionally substituted 3-8 membered heterocyclic ring, or optionally substituted C₃-C₈ cycloalkyl, and a substituent is selected from halogen, C₁-C₈ linear or branched alkyl, C₁-C₈ alkoxy, and haloalkyl.

In one preferred embodiment, the heteroaryl is 5-10 membered monocyclic or bicyclic heteroaryl containing 1-5 heteroatoms independently selected from nitrogen, oxygen, or sulfur, including pyridyl, pyridazinyl, triazinyl, pyrimidinyl, thienyl, furanyl, oxazolyl, thiazolyl, thiadiazolyl, oxadiazolyl, isoxazolyl, pyrazolyl, imidazolyl, pyrrolyl, pyranyl, pyridazinyl, pyrazinyl, and triazolyl. In another preferred embodiment, the aromatic ring is phenyl. In yet another preferred embodiment, the 3-8 membered heterocyclic ring is a 3-6 membered heterocyclic ring. In yet another preferred embodiment, the C₃-C₈ cycloalkyl is C₃-C₆ cycloalkyl. In one specific embodiment, the heteroaryl is pyridyl, pyridazinyl, pyrimidinyl, thienyl, furanyl, oxazolyl, thiazolyl, pyrazolyl, imidazolyl, pyrrolyl, and pyranyl. In one more specific embodiment, the heteroaryl is pyridyl, pyrimidinyl, thienyl, furanyl, oxazolyl, or thiazolyl.

In one embodiment, the C₃-C₈ cycloalkyl is C₃-C₆ cycloalkyl. In one preferred embodiment, the C₃-C₈ cycloalkyl and the C₃-C₆ cycloalkyl are cyclopropyl, cyclobutyl, cyclopentenyl, cyclopentyl, and cyclohexyl. In one more preferred embodiment, the C₃-C₈ cycloalkyl and the C₃-C₆ cycloalkyl are cyclopropyl, cyclopentenyl, cyclopentyl, and cyclohexyl. In one particularly preferred embodiment, the C₃-C₈ cycloalkyl and the C₃-C₆ cycloalkyl are cyclopentenyl.

In one embodiment, Q, W, Y, and U are independently selected from C and N, and Y and Q are not simultaneously C. In one preferred embodiment, Y is N, and Q is C. In one preferred embodiment, Y is C, and Q is N. In another preferred embodiment, Y is N, and Q is N.

In one embodiment, the C₁-C₈ linear or branched alkyl is selected from C₁-C₅ linear or branched alkyl. In one specific embodiment, the C₁-C₈ linear or branched alkyl and the C₁-C₅ linear or branched alkyl are each independently selected from methyl, ethyl, propyl, isopropyl, butyl, isobutyl, pentyl, and isopentyl. In one more specific embodiment, the C₁-C₈ linear or branched alkyl and the C₁-C₅ linear or branched alkyl are each independently selected from methyl, ethyl, propyl, and isopropyl.

In one embodiment, the propyl includes, but is not limited to, n-propyl (n-Pr, -CH₂CH₂CH₃) or isopropyl (i-Pr, -CH(CH₃)₂). The butyl includes, but is not limited to, n-butyl (n-Bu, -CH₂CH₂CH₂CH₃), isobutyl (i-Bu, -CH₂CH(CH₃)₂), sec-butyl (s-Bu, -CH(CH₃)CH₂CH₃), or tert-butyl (t-Bu, -C(CH₃)₃). The pentyl includes, but is not limited to, n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), or 2-methyl-1-butyl (-CH₂CH(CH₃)CH₂CH₃).

In one embodiment, the C₁-C₈ alkoxy is selected from C₁-C₅ alkoxy. In one specific embodiment, the C₁-C₈ alkoxy and the C₁-C₅ alkoxy are each independently selected from methoxy, ethoxy, propoxy, butoxy, and pentyloxy. In one more specific embodiment, the C₁-C₈ alkoxy and the C₁-C₅ alkoxy are each independently selected from methoxy, ethoxy, and propoxy.

In one embodiment, the halogen is fluorine, chlorine, bromine, or iodine. In one preferred embodiment, the halogen is fluorine, chlorine, or bromine. In one more preferred embodiment, the halogen is fluorine or chlorine. In one particularly preferred embodiment, the halogen is fluorine.

In one specific embodiment, the compound of formula I is a compound represented by formula V: 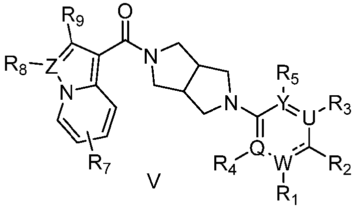
wherein R₁, R₂, R₃, R₄, and R₅ are absent or independently selected from H, fluorine, chlorine, methyl, ethyl, propyl, isopropyl, methoxy, ethoxy, and propoxy;
or R₁ and R₂ form C₃-C₆ cycloalkyl, 6-10 membered monocyclic or bicyclic aryl, 5-10 membered monocyclic or bicyclic heteroaryl containing 1-5 heteroatoms independently selected from nitrogen, oxygen, or sulfur, or a 3-8 membered monocyclic or bicyclic heterocyclic ring containing 1-3 heteroatoms independently selected from nitrogen, oxygen, or sulfur;
Q, W, Y, and U are independently selected from C and N, and Y and Q are not simultaneously C;
--- is a single bond or a double bond;
Z is selected from C and N;
R₇ is selected from H, fluorine, chlorine, methyl, ethyl, propyl, and isopropyl;
R₈ is absent or independently selected from H, fluorine, chlorine, methyl, ethyl, propyl, and isopropyl;
R₉ is optionally substituted 5-10 membered monocyclic or bicyclic heteroaryl containing 1-5 heteroatoms independently selected from nitrogen, oxygen, or sulfur, an optionally substituted 6-10 membered monocyclic or bicyclic aromatic ring group, an optionally substituted 3-8 membered monocyclic or bicyclic heterocyclic ring containing 1-3 heteroatoms independently selected from nitrogen, oxygen, or sulfur, or optionally substituted C₃-C₈ cycloalkyl, and a substituent is selected from fluorine, chlorine, methyl, ethyl, propyl, isopropyl, methoxy, ethoxy, propoxy, trifluoromethyl, fluoromethyl, and difluoromethyl.

In one specific embodiment, the compound of formula I is a compound represented by formula V-1: 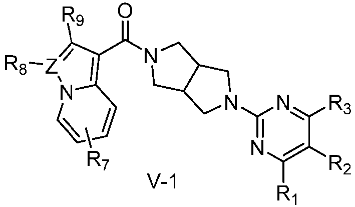
wherein R₁, R₂, and R₃ are independently selected from H, fluorine, chlorine, methyl, ethyl, propyl, isopropyl, methoxy, ethoxy, and propoxy;
or R₁ and R₂ form C₃-C₆ cycloalkyl 6-10 membered monocyclic or bicyclic aryl, 5-10 membered monocyclic or bicyclic heteroaryl containing 1-5 heteroatoms independently selected from nitrogen, oxygen, or sulfur, or a 3-8 membered monocyclic or bicyclic heterocyclic ring containing 1-3 heteroatoms independently selected from nitrogen, oxygen, or sulfur;
Z is selected from C and N;
R₇ is selected from H, fluorine, chlorine, methyl, ethyl, propyl, and isopropyl;
R₈ is absent or independently selected from H, fluorine, chlorine, methyl, ethyl, propyl, and isopropyl;
R₉ is optionally substituted 5-10 membered monocyclic or bicyclic heteroaryl containing 1-5 heteroatoms independently selected from nitrogen, oxygen, or sulfur, an optionally substituted 6-10 membered monocyclic or bicyclic aromatic ring group, an optionally substituted 3-8 membered monocyclic or bicyclic heterocyclic ring containing 1-3 heteroatoms independently selected from nitrogen, oxygen, or sulfur, or optionally substituted C₃-C₈ cycloalkyl; the substituent is selected from fluorine, chlorine, methyl, ethyl, propyl, isopropyl, methoxy, ethoxy, propoxy, trifluoromethyl, fluoromethyl, and difluoromethyl.

In one specific embodiment, the compound of formula I is a compound represented by formula VI: 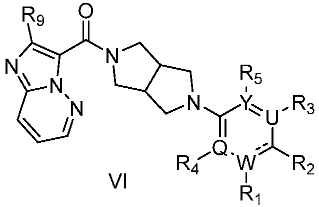
wherein R₁, R₂, R₃, R₄, and R₅ are absent or independently selected from H, fluorine, chlorine, methyl, ethyl, propyl, isopropyl, methoxy, ethoxy, and propoxy;
or R₁ and R₂ form C₃-C₆ cycloalkyl, 6-10 membered monocyclic or bicyclic aryl, 5-10 membered monocyclic or bicyclic heteroaryl containing 1-5 heteroatoms independently selected from nitrogen, oxygen, or sulfur, or a 3-8 membered monocyclic or bicyclic heterocyclic ring containing 1-3 heteroatoms independently selected from nitrogen, oxygen, or sulfur;
Q, W, Y, and U are independently selected from C and N, and Y and Q are not simultaneously C;
--- is a single bond or a double bond;
R₉ is optionally substituted 5-10 membered monocyclic or bicyclic heteroaryl containing 1-5 heteroatoms independently selected from nitrogen, oxygen, or sulfur, an optionally substituted 6-10 membered monocyclic or bicyclic aromatic ring group, an optionally substituted 3-8 membered monocyclic or bicyclic heterocyclic ring containing 1-3 heteroatoms independently selected from nitrogen, oxygen, or sulfur, or optionally substituted C₃-C₈ cycloalkyl; the substituent is selected from fluorine, chlorine, methyl, ethyl, propyl, isopropyl, methoxy, ethoxy, propoxy, trifluoromethyl, fluoromethyl, and difluoromethyl.

In one specific embodiment, the compound of formula I is a compound represented by formula VII: 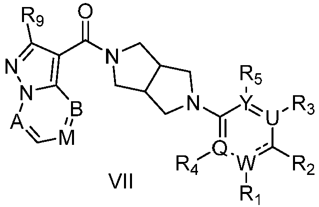
wherein: R₁, R₂, R₃, R₄, and R₅ are absent or independently selected from H, fluorine, chlorine, methyl, ethyl, propyl, isopropyl, methoxy, ethoxy, and propoxy;
or R₁ and R₂ form C₃-C₆ cycloalkyl, 6-10 membered monocyclic or bicyclic aryl, 5-10 membered monocyclic or bicyclic heteroaryl containing 1-5 heteroatoms independently selected from nitrogen, oxygen, or sulfur, or a 3-8 membered monocyclic or bicyclic heterocyclic ring containing 1-3 heteroatoms independently selected from nitrogen, oxygen, or sulfur;
Q, W, Y, and U are independently selected from C and N, and Y and Q are not simultaneously C;
--- is a single bond or a double bond;
A, B, and M are selected from CH and N, and A, B, and M are not simultaneously CH;
R₉ is optionally substituted 5-10 membered monocyclic or bicyclic heteroaryl containing 1-5 heteroatoms independently selected from nitrogen, oxygen, or sulfur, an optionally substituted 6-10 membered monocyclic or bicyclic aromatic ring group, an optionally substituted 3-8 membered monocyclic or bicyclic heterocyclic ring containing 1-3 heteroatoms independently selected from nitrogen, oxygen, or sulfur, or optionally substituted C₃-C₈ cycloalkyl; the substituent is selected from fluorine, chlorine, methyl, ethyl, propyl, isopropyl, methoxy, ethoxy, propoxy, trifluoromethyl, fluoromethyl, and difluoromethyl.

In one specific embodiment, the compound represented by formula I is selected from any one of the following compounds: 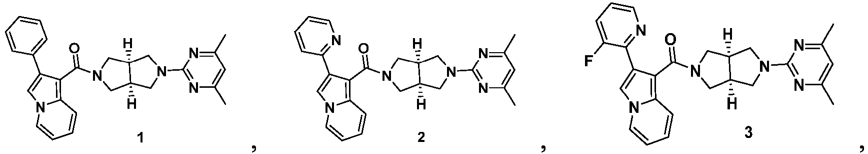 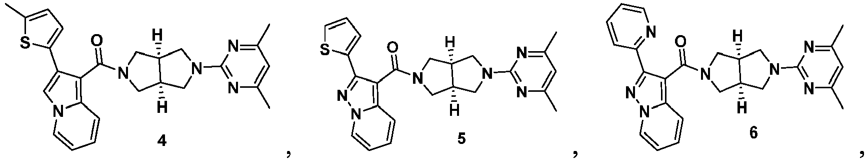 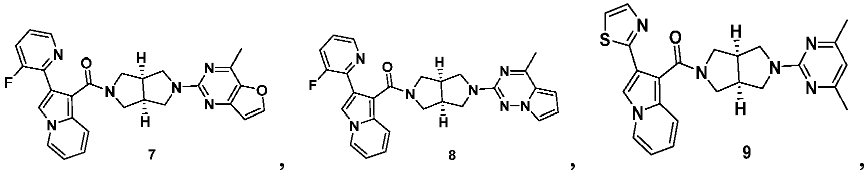 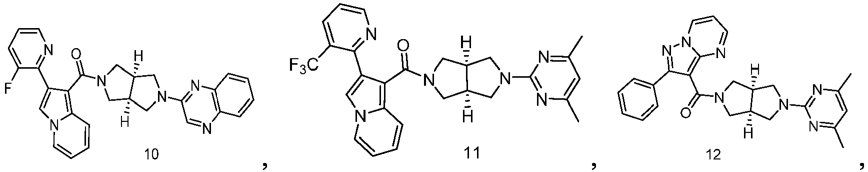 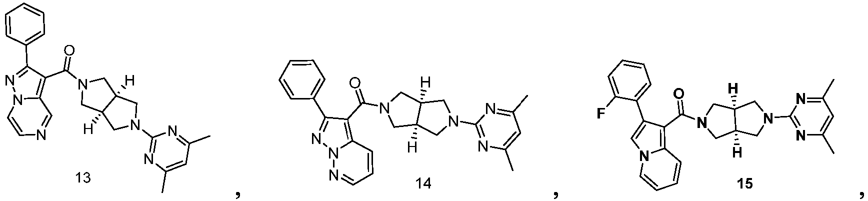 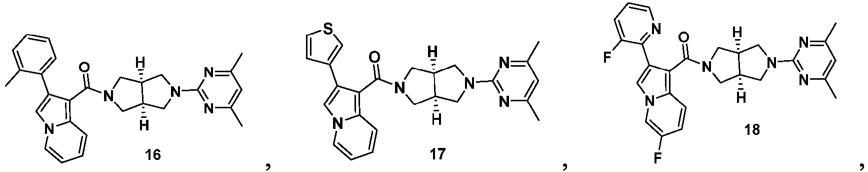 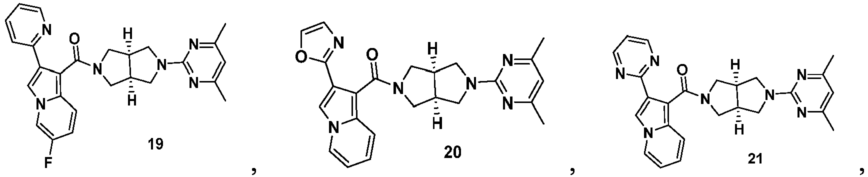 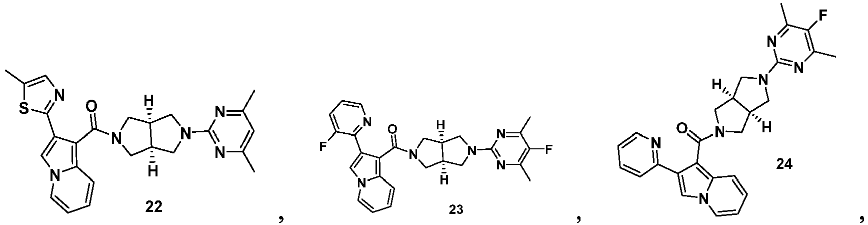 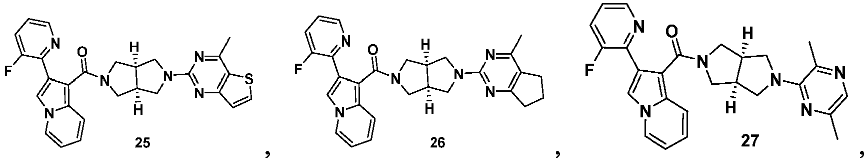 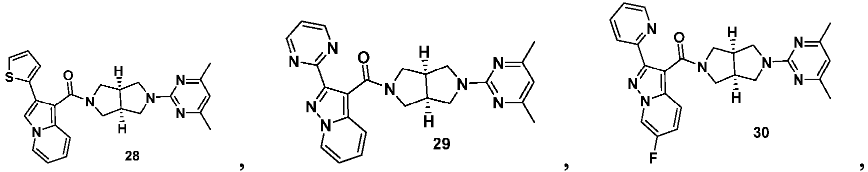 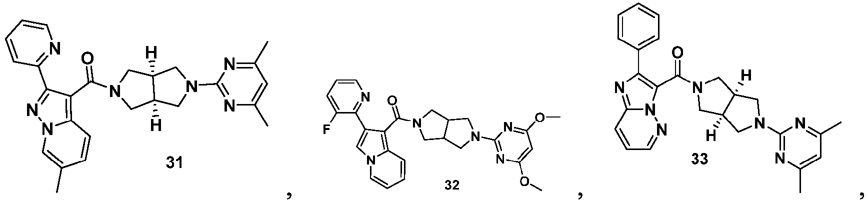 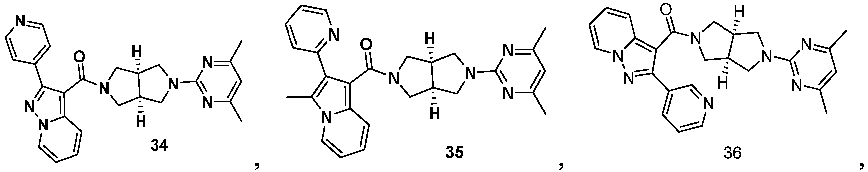 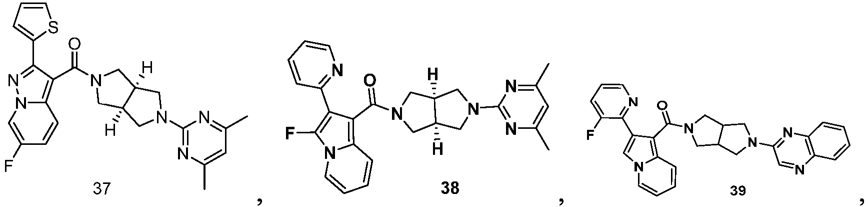 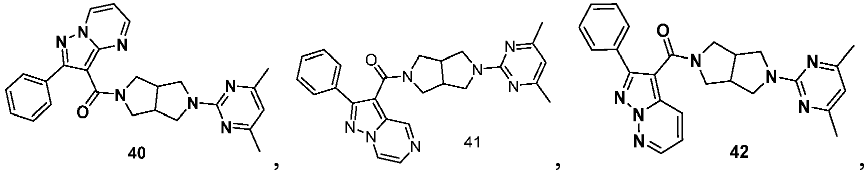 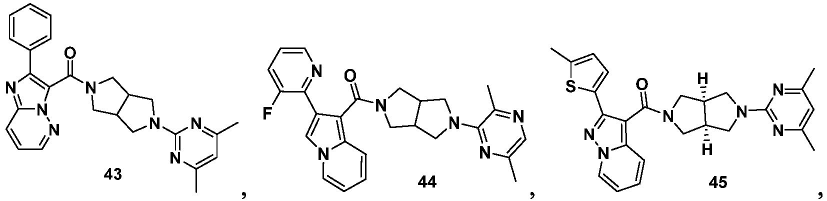 and

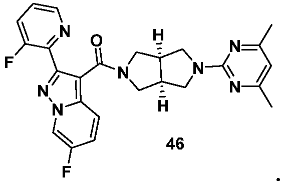

### Beneficial Technical Effects of the Present Invention

Compared with the prior art, the technical solutions of the present invention have the following advantages:
The compounds provided by the present invention have relatively good selectivity when acting on OX1R and OX2R. The compounds provided by the present invention are used for treating a sleep disorder-related disease. The compounds provided by the present invention have relatively good pharmacodynamic activity, as well as excellent physicochemical properties and pharmacokinetic properties.

### Examples

Examples of the present invention are described in detail below. The examples described below are exemplary and are intended to explain the present invention only, and they should not be construed as limiting the present invention. Unless otherwise indicated, the proportions, percentages, and the like referred to herein are calculated by weight.

### Synthetic Examples

### Example 1: ((3aR,6aS)-5-(4,6-dimethylpyrimidin-2-yl)hexahydropyrrolo[3,4-c]pyrrol-2(1H)-yl)(2-phenylindolizin-1-yl)methanone

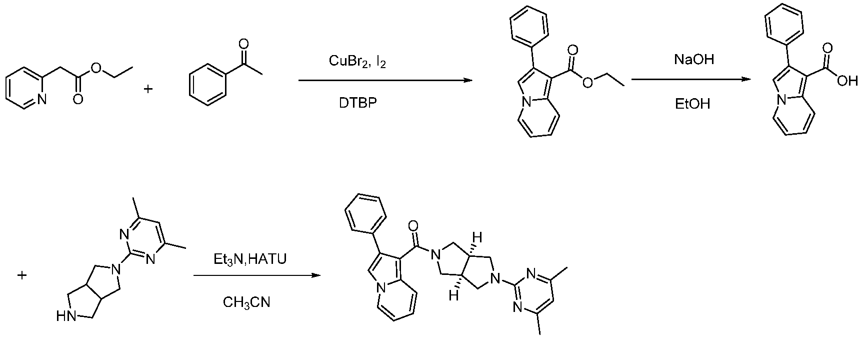

### 1.1. Preparation of ethyl 2-phenylindolizine-1-carboxylate

Ethyl 2-pyridylacetate (1.0 g, 6.06 mmol), acetophenone (1.45 g, 12.11 mmol), CuBr₂ (0.14 g, 0.61 mmol), I₂ (0.31 g, 1.21 mmol), and DTBP (0.89 g, 6.06 mmol) were added to a 50 mL three-necked round-bottom flask and left to react overnight at 78 °C under N₂ atmosphere. After TLC analysis showed the completion of the reaction, the reaction mixture was cooled to room temperature, quenched with a saturated aqueous Na₂S₂O₃ solution, extracted with 3 × 30 mL of EA, washed with brine, dried over anhydrous sodium sulfate, and then concentrated to give a crude product. The crude product was subjected to ethyl acetate/petroleum ether (1/3) silica gel column chromatography to give 0.8 g as a tawny oily liquid; LCMS (ES, m/z): 266 [M+H]⁺.

### 1.2. Preparation of 2-phenylindolizine-1-carboxylic acid

Ethyl 2-phenylindolizine-1-carboxylate (0.74 g, 2.81 mmol), sodium hydroxide (0.56 g, 14.07 mmol), and EtOH/H₂O (10/2 mL) were added to a 100 mL single-necked round-bottom flask and left to react overnight at 65 °C. After TLC analysis showed the completion of the reaction, the reaction mixture was cooled to room temperature and concentrated to remove ethanol. Water was then added to the residue, and the pH was adjusted to 4-5 with 3 N HCl. The mixture was extracted with 3 × 30 mL of EA, and the extract was then washed with 30 mL of saturated brine, dried over anhydrous sodium sulfate, filtered under vacuum, and concentrated to give 0.65 g of a pale yellow solid; LCMS (ES, m/z): 238 [M+H]⁺.

### 1.3. Preparation of ((3aR,6aS)-5-(4,6-dimethylpyrimidin-2-yl)hexahydropyrrolo [3,4-c]pyrrol-2(1H)-yl)(2-phenylindolizin-1-yl)methanone

2-Phenylindolizine-1-carboxylic acid (510 mg, 2.15 mmol), HATU (1.63 g, 4.29 mmol), Et₃N (652 mg, 6.44 mmol), and acetonitrile (10 mL) were added to a 50 mL single-necked round-bottom flask under an ice bath and left to react for 10 min at that temperature. 2-(4,6-Dimethylpyrimidin-2-yl)octahydropyrrolo[3,4-c]pyrrole (562 mg, 2.58 mmol) was added, and the mixture was left to react for 2 h at room temperature. After TLC analysis showed the completion of the reaction, 30 mL of water was added, and extraction was performed with 3 × 30 mL of DCM. The extract was then washed with 30 mL of saturated brine, dried over anhydrous sodium sulfate, filtered under vacuum, concentrated, and subjected to DCM/MeOH (20/1) silica gel column chromatography to give 585 mg of the product. ¹H NMR (400 MHz, Chloroform-d) δ 8.51 (dd, J = 7.5, 1.4 Hz, 1H), 7.67 (td, J = 7.5, 1.4 Hz, 1H), 7.54 - 7.49 (m, 3H), 7.49 - 7.34 (m, 4H), 6.74 (s, 1H), 6.68 (td, J = 7.5, 1.5 Hz, 1H), 4.04-3.99 (m, 2H), 3.79 - 3.58 (m, 4H), 3.38-3.33 (m, 2H), 2.80 - 2.62 (m, 2H), 2.25 (s, 6H); LCMS (ES, m/z): 438 [M+H]⁺.

### Example 2: ((3aR,6aS)-5-(4,6-dimethylpyrimidin-2-yl)hexahydropyrrolo[3,4-c]pyrrol-2(1H)-yl)(2-(pyridin-2-yl)indolizin-1-yl)methanone

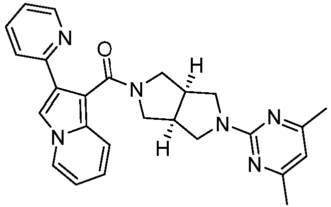

The starting material of the reaction, acetophenone, was replaced with 1-(pyridin-2-yl)ethan-1-one, and the method of Example 1 was followed to prepare the target compound. ¹H NMR (400 MHz, Chloroform-d) δ 8.62 (d, *J* = 4.4 Hz, 1H), 7.97 (d, *J* = 6.8 Hz, 1H), 7.80 (s, 1H), 7.70 - 7.59 (m, 2H), 7.55 (d, *J* = 9.1 Hz, 1H), 7.07 (s, 1H), 6.91 - 6.78 (m, 1H), 6.63 (t, *J =* 6.8 Hz, 1H), 6.34 (s, 1H), 4.23 - 3.19 (m, 8H), 3.02-2.84 (m, 2H), 2.35 (s, 6H). LCMS (ES, m/z): 439 [M+H]⁺.

### Example 3: ((3aR,6aS)-5-(4,6-dimethylpyrimidin-2-yl)hexahydropyrrolo[3,4-c]pyrrol-2(1H)-yl)(2-(3-fluoropyridin-2-yl)indolizin-1-yl)methanone

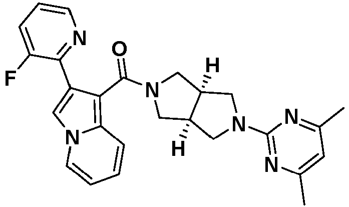

The starting material of the reaction, acetophenone, was replaced with 1-(3-fluoropyridin-2-yl)ethan-1-one, and the method of Example 1 was followed to prepare the target compound. ¹H NMR (400 MHz, CDCl₃) δ 8.41 (d, *J* = 4.6 Hz, 1H), 7.91 (d, *J =* 6.9 Hz, 1H), 7.79 (d, *J =* 2.4 Hz, 1H), 7.44 (d, *J =* 9.1 Hz, 1H), 7.37 (m, 1H), 7.05 (m, 1H), 6.77 (dd, *J* = 9.1, 6.5 Hz, 1H), 6.56 (t, *J* = 6.7 Hz, 1H), 6.29 (s, 1H), 3.94 - 3.47 (m, 7H), 3.07 - 2.88 (m, 3H), 2.32 (s, 6H). LCMS (ES, m/z): 457 [M+H]⁺.

### Example 4: ((3aR,6aS)-5-(4,6-dimethylpyrimidin-2-yl)hexahydropyrrolo[3,4-c]pyrrol-2(1H)-yl)(2-(5-methylthiophen-2-yl)indolizin-1-yl)methanone

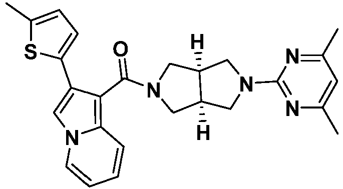

The starting material of the reaction, acetophenone, was replaced with 1-(5-methylthiophen-2-yl)ethan-1-one, and the method of Example 1 was followed to prepare the target compound. ¹H NMR (400 MHz, Chloroform-d) δ 8.51 (dd, *J* = 7.5, 1.4 Hz, 1H), 7.83 (s, 1H), 7.69 - 7.65(m, 1H), 7.44 (dd, *J* = 7.5, 1.3 Hz, 1H), 7.28 (d, *J* = 7.6 Hz, 1H), 6.74 (s, 1H), 6.70 - 6.66 (m, 2H), 4.16 - 3.27 (m, 8H), 2.79 - 2.57 (m, 2H), 2.44 (s, 3H), 2.25 (s, 6H). LCMS (ES, m/z): 458 [M+H]⁺.

### Example 5: ((3aR,6aS)-5-(4,6-dimethylpyrimidin-2-yl)hexahydropyrrolo[3,4-c]pyrrol-2(1H)-yl)(2-(thiophen-2-yl)pyrazolo[1,5-a]pyridin-3-yl)methanone

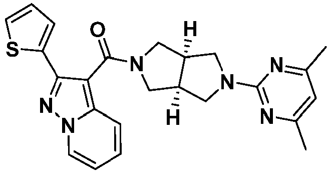

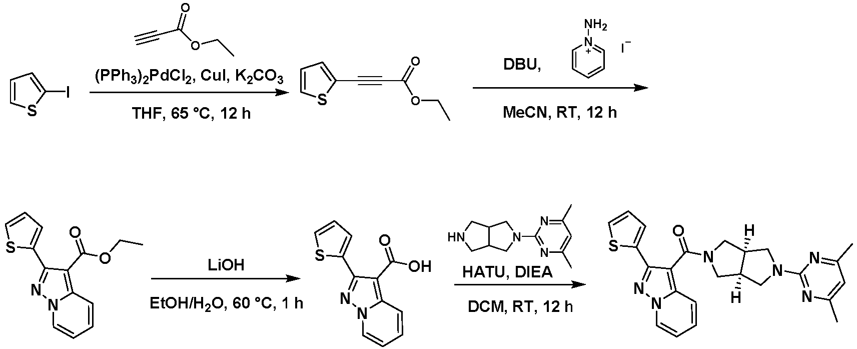

### 5.1. Preparation of ethyl 3-(thiophen-2-yl)propiolate

2-Iodothiophene (2.0 g, 9.52 mmol), ethyl propiolate (3.74 g, 38.09 mmol), (PPh₃)₂PdCl₂ (134 mg, 0.19 mmol), CuI (73 mg, 0.38 mmol), and K₂CO₃ (2.63 g, 19.04 mmol) were added to a 100 mL single-necked flask containing THF (40 mL), and N₂ was introduced. The mixture was stirred for reaction for 12 h at 65 °C. The reaction mixture was filtered, and the filtrate was concentrated and then subjected to silica gel column chromatography (EA:PE = 0-10%) to give 1.5 g of a pale yellow oil.

### 5.2. Preparation of ethyl 2(thiophen-2-yl)pyrazolo[1,5-a]pyridine-3-carboxylate

Ethyl 3-(thiophen-2-yl)propiolate (500 mg, 2.77 mmol), 1-aminopyridinium iodide (616 mg, 2.77 mmol), and DBU (845 mg, 5.55 mmol) were added to a 100 mL single-necked flask containing MeCN (20 mL), and N₂ was introduced. The mixture was left to react overnight at room temperature. The reaction mixture was concentrated and extracted with EA (3 × 30 mL). The organic phase was washed with saturated brine, dried over anhydrous sodium sulfate, concentrated, and subjected to silica gel column chromatography (EA:PE = 0-20%) to give 500 mg of a pale yellow solid.

### 5.3. Preparation of 2-(thiophen-2-yl)pyrazolo[1,5-a]pyridine-3-carboxylic acid

Ethyl 2-(thiophen-2-yl)pyrazolo[1,5-a]pyridine-3-carboxylate (500 mg, 1.84 mmol), LiOH (88 mg, 3.67 mmol), and EtOH/H₂O (6/3 mL) were added to a 50 mL single-necked flask and stirred for reaction for 1 h at 60 °C. The reaction mixture was concentrated, the pH was then adjusted to 5-6 with 1 M HCl, and extraction was performed with EA (3 × 30 mL). The extract was then washed with saturated brine, dried over anhydrous sodium sulfate, and concentrated to give 400 mg of a white solid.

### 5.4. Preparation of ((3aR,6aS)-5-(4,6-dimethylpyrimidin-2-yl)hexahydropyrrolo [3,4-c]pyrrol-2(1H)-yl)(2-(thiophen-2-yl)pyrazolo[1,5-a]pyridin-3-yl)methanone

2-(Thiophen-2-yl)pyrazolo[1,5-a]pyridine-3-carboxylic acid (100 mg, 0.41 mmol) was added to a single-necked flask containing 5 mL of DCM, and HATU (171 mg, 0.45 mmol) and DIEA (159 mg, 1.23 mmol) were added. After 10 min of stirring, (3aR,6aS)-2-(4,6-dimethylpyrimidin-2-yl)octahydropyrrolo[3,4-c]pyrrole (98 mg, 0.45 mmol) was added. The mixture was left to react overnight at room temperature. The reaction mixture was concentrated and subjected to silica gel column chromatography (EA:PE = 0-100%) to give 80 mg of the product. ¹H NMR (400 MHz, Methanol-*d*₄) δ 8.46 - 8.39 (m, 2H), 7.35 - 7.19 (m, 1H), 7.18 - 7.05 (m, 2H), 6.98 - 6.87 (m, 1H), 6.85 - 6.61 (m, 1H), 6.42 (s, 1H), 3.52 - 3.47 (m, 1H), 3.39 - 3.33 (m, 1H), 4.01 - 3.81 (m, 2H), 3.75 - 3.66 (m, 1H), 3.63 - 3.56 (m, 1H), 3.53 - 3.47 (m, 1H), 3.45 - 3.39 (m, 1H), 3.26 - 3.19 (m, 1H), 3.16 - 3.11 (m, 1H), 3.07 - 2.91 (m, 2H), 2.29 (d, J = 2.8 Hz, 6H),. LCMS (ES, m/z): 445 [M+H]⁺.

### Example 6: ((3aR,6aS)-5-(4,6-dimethylpyrimidin-2-yl)hexahydropyrrolo[3,4-c]pyrrol-2(1H)-yl)(2-(pyridin-2-yl)pyrazolo[1,5-a]pyridin-3-yl)methanone

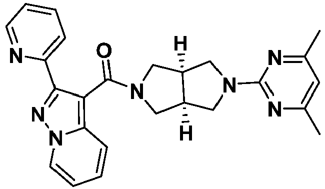

The starting material of the reaction, 2-iodothiophene, was replaced with 2-iodopyridine, and the method of Example 1 was followed to prepare the target compound.

¹H NMR (400 MHz, Chloroform-d) δ 8.76 - 8.52 (m, 1H), 7.73 - 7.53 (m, 1H), 7.46 - 7.23 (m, 2H), 7.18 - 7.12 (m, 1H), 7.08- 6.91 (m, 3H), 6.53 (s, 1H), 3.52-3.11(m, 8H), 3.07 - 2.91 (m, 2H), 2.29 (d, J = 2.8 Hz, 6H),. LCMS (ES, m/z): 440 [M+H]⁺.

### Example 7: (2-(3-fluoropyridin-2-yl)indolizin-1-yl)((3aR,6aS)-5-(4-methylfuro [3,2-d]pyrimidin-2-yl)hexahydropyrrolo[3,4-c]pyrrol-2(1H)-yl)methanone

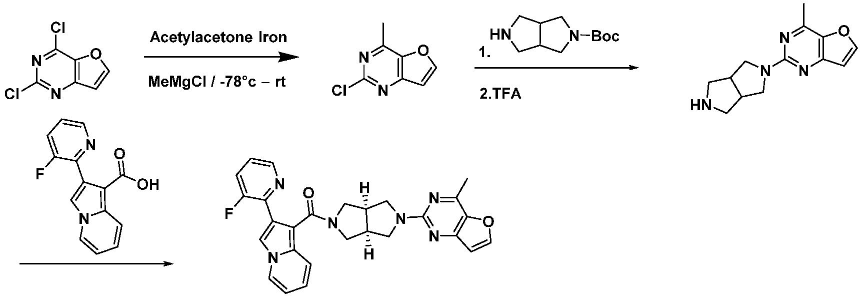

### 7.1. Preparation of 2-chloro-4-methylfuro[3,2-d]pyrimidine

2,4-Dichlorofuro[3,2-d]pyrimidine (3.0 g, 15.8 mmol), acetylacetone iron (0.22 g, 0.9 mmol), and tetrahydrofuran (30 mL) were placed in a 100 mL round-bottom flask, and methylmagnesium chloride (3 N, 10.5 mL, 31.6 mmol) was added dropwise to the flask at -78 °C. After the dropwise addition was complete, the mixture was stirred for one hour at that temperature and then stirred at room temperature. After TLC monitoring showed the completion of the reaction, the reaction mixture was quenched by adding 20 mL of water and extracted with ethyl acetate three times (30 mL × 3). The organic phase was washed with 20 mL of saturated brine, dried over anhydrous sodium sulfate, filtered under vacuum, concentrated, and separated by column chromatography (petroleum ether/ethyl acetate = 3:1) to give 2.2 g of the product as a white solid.

### 7.2. Preparation of 2-(hexahydropyrrolo[3,4-c]pyrrol-2(1H)-yl)-4-methylfuro[3,2-d]pyrimidine

2-Chloro-4-methylfuro[3,2-d]pyrimidine (0.25 g, 1.5 mmol), tert-butyl hexahydropyrrolo[3,4-c]pyrrole-2(1H)-carboxylate (0.42 g, 2.0 mmol), and N-methylpyrrolidone (2 mL) were placed in a 10 mL three-necked flask and left to react for 1.5 h at 170 °C in a microwave reactor. The reaction mixture was cooled to room temperature, then diluted by adding 10 mL of water and 10 mL of ethyl acetate, and then filtered through diatomaceous earth. The filtrate was extracted with ethyl acetate three times (20 mL × 3). The organic phase was washed with 20 mL of saturated brine, dried over anhydrous sodium sulfate, filtered under vacuum, concentrated, and separated by column chromatography (petroleum ether/ethyl acetate = 1:3) to give a pale yellow oil. The oil was dissolved in dichloromethane, and trifluoroacetic acid was added. After 4 h of reaction, the organic phase was washed with a saturated sodium carbonate solution and dried, and the solvent was evaporated to dryness to give 0.11 g of the product as a yellow oil.

### 7.3. Preparation of (2-(3-fluoropyridin-2-yl)indolizin-1-yl)((3aR,6aS)-5-(4-methylfuro [3,2-d] pyrimidi n-2-yl)hexahydropyrrolo[3,4-c]pyrrol-2(1H)-yl)methanone

2-(3-Fluoropyridin-2-yl)indolizine-1-carboxylic acid (0.38 g, 1.5 mmol) (Example 3), HATU (0.76 g, 2.0 mmol), DIEA (0.39 g, 3.0 mmol), and dichloromethane (20 mL) were placed in a 100 mL round-bottom flask. After 20 min of stirring at room temperature, 2-(hexahydropyrrolo[3,4-c]pyrrol-2(1H)-yl)-4-methylfuro [3,2-d]pyrimidine (0.29 g, 1.2 mmol) was gradually added in portions, and continue stirring at room temperature overnight. After TLC monitoring showed the completion of the reaction, the reaction mixture was directly concentrated and then separated by column chromatography (methanol/dichloromethane = 1:30) to give 0.45 g of the product. ¹H NMR (400 MHz, Chloroform-d) δ8.51 (dd, *J =* 14.9, 3.1 Hz, 1H), 8.36 (dd, *J =* 14.9, 3.1 Hz, 1H), 7.77 - 7.54 (m, 2H), 7.52 - 7.34 (m, 4H), 7.24 (d, *J* = 14.9 Hz, 1H), 6.72-6.38 (m, 1H), 4.12 (q, J = 7.1 Hz, 1H), 4.04 - 3.88 (m, 2H), 3.76 (ddd, J = 24.6, 13.2, 5.3 Hz, 2H), 3.70 - 3.55 (m, 2H), 3.27 (td, J = 10.6, 5.2 Hz, 1H), 3.20 - 2.96 (m, 2H), 2.58 (s, 3H);. LCMS (ES, m/z): 483 [M+H]⁺.

### Example 8: (2-(3-Fluoropyridin-2-yl)indolizin-1-yl)((3aR,6aS)-5-(4-methylpyrrolo [2,1-f][1,2,4]triazin-2-yl)hexahydropyrrolo[3,4-c]pyrrol-2(1H)-yl)methanone

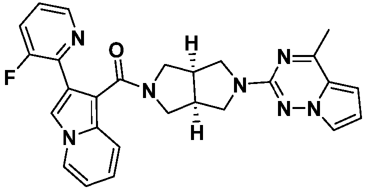

The starting material of the reaction, 2,4-dichlorofuro[3,2-d]pyrimidine, was replaced with 2,4-dichloropyrrolo[2,1-f][1,2,4]triazine, and the method of Example 7 was followed to prepare the target compound. ¹H NMR (400 MHz, Chloroform) δ 8.44 (dd, *J =* 14.9, 3.1 Hz, 1H), 8.31 (dd, *J =* 14.9, 3.1 Hz, 1H), 7.74 - 7.55 (m, 2H), 7.51 - 7.31 (m, 4H), 6.72-6.64 (m, 1H), 6.57 (t, *J* = 7.5 Hz, 1H), 6.36 (dd, *J* = 7.5, 1.5 Hz, 1H), 4.29 - 4.03 (m, 2H), 3.93 - 3.61 (m, 4H), 3.57 - 3.23 (m, 2H), 2.89 - 2.57 (m, 5H). LCMS (ES, m/z): 482 [M+H]⁺.

### Example 9: ((3aR,6aS)-5-(4,6-dimethylpyrimidin-2-yl)hexahydropyrrolo[3,4-c]pyrrol-2(1H)-yl)(2-(thiazol-2-yl)indolizin-1-yl)methanone

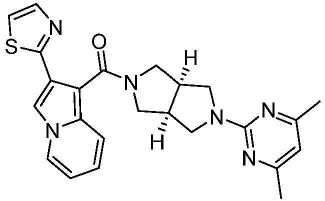

The starting material of the reaction, acetophenone, was replaced with 2-acetylthiazole, and the method of Example 1 was followed to prepare the target compound. ¹H NMR (400 MHz, CDCl₃) δ 7.90 (d, *J* = 7.0 Hz, 1H), 7.82 - 7.72 (m, 2H), 7.43 (d, *J =* 9.1 Hz, 1H), 7.22 (d, *J =* 3.2 Hz, 1H), 6.81 (m, 1H), 6.60 (t, *J =* 6.7 Hz, 1H), 6.29 (s, 1H), 3.96 (d, *J* = 57.8 Hz, 2H), 3.69 (d, *J* = 58.0 Hz, 3H), 3.44 (s, 2H), 3.07 (d, *J* = 35.3 Hz, 2H), 2.88 (s, 1H), 2.31 (s, 6H). LCMS (ES, m/z): 445 [M+H]⁺.

### Example 10: (2-(3-Fluoropyridin-2-yl)indolizin-1-yl)((3aR,6aS)-5-(quinoxalin-2-yl)hexahydrop yrrolo[3,4-c]pyrrol-2(1H)-yl)methanone

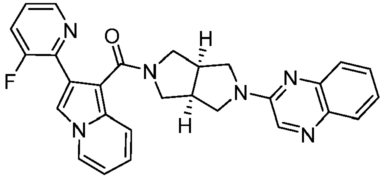

The starting material of the reaction, 2,4-dichlorofuro[3,2-d]pyrimidine, was replaced with 2-chloroquinoxaline, and the method of Example 7 was followed to prepare the target compound. ¹H NMR (400 MHz, CDCl₃) δ 8.35 (d, *J* = 4.6 Hz, 1H), 8.31 (s, 1H), 7.91 (t, *J =* 6.8 Hz, 2H), 7.78 (d, *J =* 2.5 Hz, 1H), 7.60 (t, *J =* 7.5 Hz, 1H), 7.49 - 7.36 (m, 2H), 7.33 (t, *J =* 9.5 Hz, 1H), 6.94 (s, 1H), 6.78 (m, 1H), 6.57 (t, *J =* 6.7 Hz, 1H), 4.06 - 3.40 (m, 8H), 3.14 (s, 2H). LCMS (ES, m/z): 479[M+H]⁺.

### Example 11: ((3aR,6aS)-5-(4,6-dimethylpyrimidin-2-yl)hexahydropyrrolo[3,4-c]pyrrol-2(1H)-yl)(2-(3-(trifluoromethyl)pyridin-2-yl)indolizin-1-yl)methanone

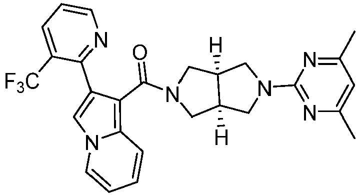

The starting material of the reaction, acetophenone, was replaced with 2-acetyl-3-trifluoromethylpyridine, and the method of Example 1 was followed to prepare the target compound. ¹H NMR (400 MHz, CDCl₃): δ 8.83 (d, *J* = 4.8 Hz, 1H), 7.98 (t, *J =* 6.4 Hz, 2H), 7.55 (d, *J =* 8.7 Hz, 2H), 7.23 (dd, *J =* 8.0, 4.8 Hz, 1H), 6.92 - 6.84 (m, 1H), 6.65 (t, *J =* 6.7 Hz, 1H), 6.36 (s, 1H), 3.77 (s, 4H), 3.49 (dd, *J =* 11.6, 3.8 Hz, 4H), 2.95 (s, 2H), 2.37 (s, 6H). LCMS (ES, m/z): 507[M+H]⁺.

### Example 12: ((3aR,6aS)-5-(4,6-dimethylpyrimidin-2-yl)hexahydropyrrolo[3,4-c]pyrrol-2(1H)-yl)(2-phenylpyrazolo [1,5-a] pyrimidin-3-yl)methanone

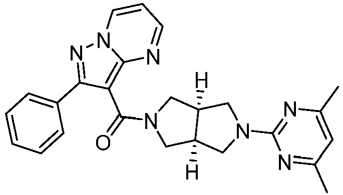

The starting materials of the reaction, 2-iodothiophene and 1-aminopyridinium iodide, were replaced with iodobenzene and 1-aminopyrimidinium iodide, respectively, and the method of Example 5 was followed to prepare the target compound. ¹H NMR (400 MHz, CDCl₃) δ 8.74 (d, *J* = 7.0, 1.7 Hz, 1H), 8.63 - 8.56 (m, 1H), 7.92 (d, *J =* 7.6 Hz, 2H), 7.46 (t, *J =* 7.5 Hz, 2H), 7.38 (t, *J =* 7.3 Hz, 1H), 6.95 (m, 1H), 6.34 (s, 1H), 4.12 (m, 1H), 3.90 (m, 1H), 3.79 (m, 1H), 3.71 (m, 1H), 3.60 (m, 2H), 3.37 (m, 1H), 3.18 (m, 1H), 3.13 - 3.02 (m, 1H), 2.92 (m, 1H), 2.34 (s, 6H). LCMS (ES, m/z): 440 [M+H]⁺.

### Example 13: ((3aR,6aS)-5-(4,6-dimethylpyrimidin-2-yl)hexahydropyrrolo[3,4-c]pyrrol-2(1H)-yl)(2-phenylpyrazolo[1,5-a]pyrazin-3-yl)methanone

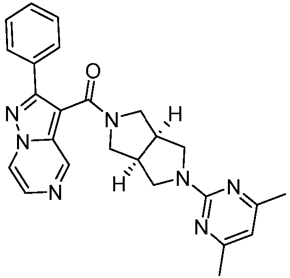

The starting materials of the reaction, 2-iodothiophene and 1-aminopyridinium iodide, were replaced with iodobenzene and 1-aminopyrazinium iodide, respectively, and the method of Example 5 was followed to prepare the target compound. ¹H NMR (400 MHz, CDCl₃) δ 9.18 (s, 1H), 8.39 (d, *J* = 4.7 Hz, 1H), 7.98 (d, *J* = 4.7 Hz, 1H), 7.76 (d, *J* = 7.9 Hz, 2H), 7.40 (t, *J* = 7.6 Hz, 2H), 7.31 - 7.18 (m, 1H), 6.30 (s, 1H), 3.96 (dd, *J =* 12.7, 7.6 Hz, 1H), 3.78 (ddd, *J =* 17.1, 12.2, 6.1 Hz, 2H), 3.56 (dd, *J =* 11.5, 7.2 Hz, 1H), 3.47 (dd, *J =* 11.6, 5.1 Hz, 1H), 3.30 - 3.09 (m, 2H), 2.97 (dd, *J =* 11.0, 4.3 Hz, 1H), 2.89 - 2.65 (m, 2H), 2.29 (s, 6H). LCMS (ES, m/z): 440[M+H]⁺.

### Example 14: ((3aR,6aS)-5-(4,6-dimethylpyrimidin-2-yl)hexahydropyrrolo[3,4-c]pyrrol-2(1H)-yl)(2-phenylpyrazolo[1,5-b]pyridazin-3-yl)methanone

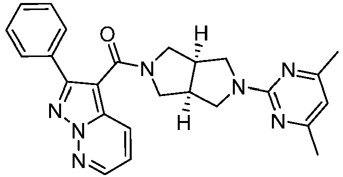

The starting materials of the reaction, 2-iodothiophene and 1-aminopyridinium iodide, were replaced with iodobenzene and 1-aminopyridazinium iodide, respectively, and the method of Example 5 was followed to prepare the target compound. ¹H NMR (400 MHz, DMSO-*d*₆) δ 8.55 (d, *J* = 4.0 Hz, 1H), 8.20 (d, *J* = 8.0 Hz, 1H), 7.74 (d, *J* = 8.0 Hz, 2H), 7.40 (t, *J =* 8.0 Hz, 2H), 7.34 - 7.25 (m, 2H), 6.38 (s, 1H), 3.87 - 3.76 (m, 1H), 3.74 - 3.64 (m, 1H), 3.61 - 3.52 (m, 1H), 3.50 - 3.40 (m, 2H), 3.32 - 3.23(m, 1H), 3.15 - 3.06 (m, 1H), 3.01 - 2.90 (m, 1H), 2.88 - 2.72 (m, 2H), 2.20 (s, 6H). LCMS (ES, m/z): 440[M+H]⁺.

### Example 15: ((3aR,6aS)-5-(4,6-dimethylpyrimidin-2-yl)hexahydropyrrolo[3,4-c]pyrrol-2(1H)-yl)(2-(2-fluorophenyl)indolizin-1-yl)methanone

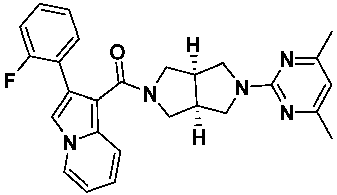

The starting material of the reaction, acetophenone, was replaced with 2-fluoroacetophenone, and the method of Example 1 was followed to prepare the target compound. ¹H NMR (400 MHz, CDCl₃) δ 7.94 (d, *J* = 6.9 Hz, 1H), 7.60 (d, *J =* 9.1 Hz, 1H), 7.49 - 7.47 (m, 1H), 7.35 (s, 1H), 7.31 - 7.29 (m, 2H), 7.13 - 7.11 (m, 1H), 6.79 (m, 1H), 6.53 - 6.52 (m, 1H), 6.28 (s, 1H), 3.85 - 3.82 (m, 2H), 3.68 -3.14 (m, 5H), 2.83 -2.65 (m, 1H), 2.29 (s, 6H). LCMS (ES, m/z): 456[M+H]⁺.

### Example 16: ((3aR,6aS)-5-(4,6-dimethylpyrimidin-2-yl)hexahydropyrrolo[3,4-c]pyrrol-2(1H)-yl)(2-(o-tolyl)indolizin-1-yl)methanone

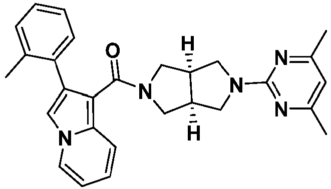

The starting material of the reaction, acetophenone, was replaced with 2-methylacetophenone, and the method of Example 1 was followed to prepare the target compound. ¹H NMR (400 MHz, CDCl₃) δ 7.86 (d, *J* = 6.9 Hz, 1H), 7.63 (d, *J =* 9.1 Hz, 1H), 7.44 - 7.42 (m, 2H), 7.30 (s, 1H), 7.30 (t, *J* = 7.6 Hz, 2H), 7.11 (d, *J =* 7.6 Hz, 1H), 6.81 (m, 1H), 6.57 (t, *J =* 6.8 Hz, 1H), 6.28 (s, 1H), 3.84 (d, *J =* 51.0 Hz, 2H), 3.62 - 3.20 (m, 5H), 2.79 - 2.61 (m, 3H), 2.29 (s, 6H), 2.17 (s, 3H). LCMS (ES, m/z): 452[M+H]⁺.

### Example 17: ((3aR,6aS)-5-(4,6-dimethylpyrimidin-2-yl)hexahydropyrrolo[3,4-c]pyrrol-2(1H)-yl)(2-(thiophen-3-yl)indolizin-1-yl)methanone

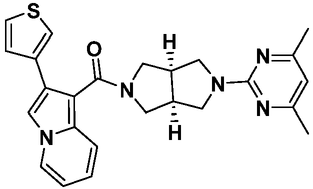

The starting material of the reaction, acetophenone, was replaced with 3-acetylthiophene, and the method of Example 1 was followed to prepare the target compound. ¹H NMR (400 MHz, CDCl₃) δ 7.80 (d, *J* = 6.6 Hz, 1H), 7.64 (s, 1H), 7.53 (d, *J =* 9.1 Hz, 1H), 7.30 (s, 1H), 7.18 - 7.15 (m, 2H), 6.73 - 6.69 (m, 1H), 6.53 - 6.51 (m, 1H), 6.25 (s, 1H), 3.93 - 3.88 (m, 2H), 3.78 - 3.43 (m, 3H), 3.26 - 2.88 (m, 5H), 2.34 (s, 6H). LCMS (ES, m/z): 443 [M+H]⁺.

### Example 18: ((3aR,6aS)-5-(4,6-dimethylpyrimidin-2-yl)hexahydropyrrolo[3,4-c]pyrrol-2(1H)-yl)(6-fluoro-2-(3-fluoropyridin-2-yl)indolizin-1-yl)methanone

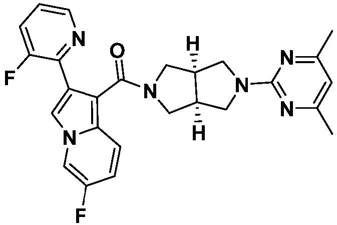

The starting materials of the reaction, acetophenone and ethyl 2-pyridylacetate, were replaced with 2-acetyl-3-fluoropyridine and ethyl 2-(5-fluoropyridin-2-yl)acetate, respectively, and the method of Example 1 was followed to prepare the target compound. ¹H NMR (400 MHz, CDCl₃) δ 8.40 (d, *J* = 4.4 Hz, 1H), 7.85 (t, *J* = 3.1 Hz, 1H), 7.78 (s, 1H), 7.48 - 7.30 (m, 2H), 7.04 (d, *J* = 7.5 Hz, 1H), 6.71 (t, *J* = 9.0 Hz, 1H), 6.28 (s, 1H), 3.97 - 3.62 (m, 5H), 3.42 - 2.86 (m, 5H), 2.29 (s, 6H). LCMS (ES, m/z): 475[M+H]⁺.

### Example 19: ((3aR,6aS)-5-(4,6-dimethylpyrimidin-2-yl)hexahydropyrrolo[3,4-c]pyrrol-2(1H)-yl)(6-fluoro-2-(pyridin-2-yl)indolizin-1-yl)methanone

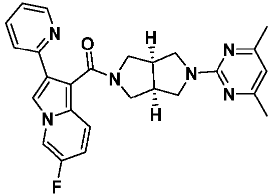

The starting materials of the reaction, acetophenone and ethyl 2-pyridylacetate, were replaced with 2-acetylpyridine and ethyl 2-(5-fluoropyridin-2-yl)acetate, respectively, and the method of Example 1 was followed to prepare the target compound. ¹H NMR (400 MHz, CDCl₃) δ 8.55 (d, *J =* 4.4 Hz, 1H), 7.79 -7.75 (m, 2H), 7.71 (s, 1H), 7.48 - 7.30 (m, 2H), 7.04 (d, *J =* 7.5 Hz, 1H), 6.76 - 6.71 (m, 1H), 6.28 (s, 1H), 3.95 - 3.67 (m, 5H), 3.46 - 2.81 (m, 5H), 2.34 (s, 6H). LCMS (ES, m/z): 457[M+H]⁺.

### Example 20: ((3aR,6aS)-5-(4,6-dimethylpyrimidin-2-yl)hexahydropyrrolo[3,4-c]pyrrol-2(1H)-yl)(2-(oxazol-2-yl)indolizin-1-yl)methanone

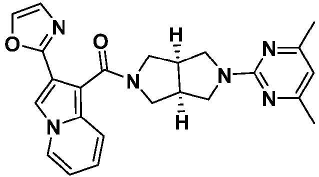

The starting material of the reaction, acetophenone, was replaced with 1-(oxazol-2-yl)ethan-1-one, and the method of Example 1 was followed to prepare the target compound. ¹H NMR (400 MHz, CDCl₃) δ 7.94 (d, *J* = 6.7 Hz, 1H), 7.86 - 7.78 (m, 2H), 7.43 (s, 1H), 7.27 (d, *J* = 3.2 Hz, 1H), 6.83 (m, 1H), 6.63 - 6.61 (m, 1H), 6.29 (s, 1H), 3.99 - 3.95 (m, 2H), 3.69 - 3.44 (m, 3H), 3.07 - 2.88 (m, 3H), 2.33 (s, 6H). LCMS (ES, m/z): 429[M+H]⁺.

### Example 21: ((3aR,6aS)-5-(4,6-dimethylpyrimidin-2-yl)hexahydropyrrolo[3,4-c]pyrrol-2(1H)-yl)(2-(pyrimidin-2-yl)indolizin-1-yl)methanone

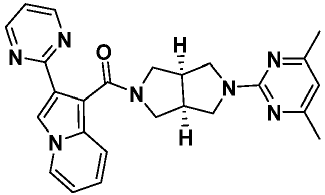

The starting material of the reaction, acetophenone, was replaced with 2-acetylpyrimidine, and the method of Example 1 was followed to prepare the target compound. ¹H NMR (400 MHz, CDCl₃) δ 8.41 (d, *J* = 4.3 Hz, 1H), 7.94 (d, *J* = 6.6 Hz, 1H), 7.78 - 7.73 (m, 2H), 7.37 (m, 1H), 7.05 (m, 1H), 6.79 -6.77 (m, 1H), 6.56 (t, *J* = 6.7 Hz, 1H), 6.29 (s, 1H), 3.94 - 3.89 (m, 2H), 3.78 - 3.47 (m, 3H), 3.07 - 2.88 (m, 2H), 2.30 (s, 6H). LCMS (ES, m/z): 440[M+H]⁺.

### Example 22: ((3aR,6aS)-5-(4,6-dimethylpyrimidin-2-yl)hexahydropyrrolo[3,4-c]pyrrol-2(1H)-yl)(2-(5-methylthiazol-2-yl)indolizin-1-yl)methanone

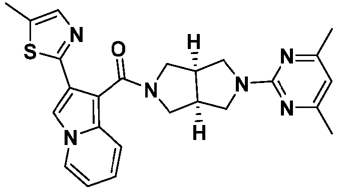

The starting material of the reaction, acetophenone, was replaced with 2-acetyl-5-methylthiazole, and the method of Example 1 was followed to prepare the target compound. ¹H NMR (400 MHz, CDCl₃) δ 7.88 (d, *J* = 6.9 Hz, 1H), 7.76 - 7.72 (m, 2H), 7.43 (d, *J =* 9.1 Hz, 1H), 6.81 (m, 1H), 6.60 (t, *J =* 6.7 Hz, 1H), 6.29 (s, 1H), 3.96 (d, *J =* 57.8 Hz, 2H), 3.63 - 3.48 (m, 4H), 3.11 - 2.93 (m, 4H), 2.46 (s, 3H), 2.34 (s, 6H). LCMS (ES, m/z): 459[M+H]⁺.

### Example 23: ((3aR,6aS)-5-(5-fluoro-4,6-dimethylpyrimidin-2-yl)hexahydropyrrolo [3,4-c]pyrrol-2(1H)-yl)(2-(3-fluoropyridin-2-yl)indolizin-1-yl)methanone

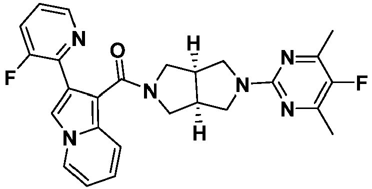

The starting material of the reaction, 2,4-dichlorofuro[3,2-d]pyrimidine, was replaced with 2,4,6-trichloro-5-fluoropyrimidine, and the method of Example 7 was followed to prepare the target compound. ¹H NMR (400 MHz, CDCl₃) δ 8.38 (d, *J* = 4.7 Hz, 1H), 7.89 (d, *J =* 6.6 Hz, 1H), 7.73 (d, *J* = 2.4 Hz, 1H), 7.49 (d, *J* = 9.1 Hz, 1H), 7.33 (m, 1H), 7.05 (m, 1H), 6.78 - 6.76 (m, 1H), 6.57 - 6.55 (m, 1H), 3.91 - 3.74 (m, 5H), 3.56 - 3.02 (m, 5H), 2.28 (s, 6H). LCMS (ES, m/z): 475[M+H]⁺.

### Example 24: ((3aR,6aS)-5-(5-fluoro-4,6-dimethylpyrimidin-2-yl)hexahydropyrrolo [3,4-c] pyrrol-2(1H)-yl)(2-(pyridin-2-yl)indolizin-1-yl)methanone

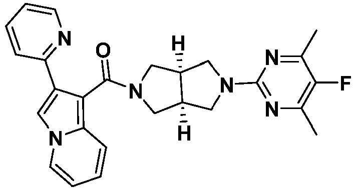

The starting materials of the reaction, 2-(3-fluoropyridin-2-yl)indolizine-1-carboxylic acid and 2,4-dichlorofuro[3,2-d]pyrimidine, were replaced with 2-(pyridin-2-yl)indolizine-1-carboxylic acid and 2,4,6-trichloro-5-fluoropyrimidine, respectively, and the method of Example 7 was followed to prepare the target compound. ¹H NMR (400 MHz, CDCl₃) δ 8.61 (d, *J* = 4.8 Hz, 1H), 7.90 (d, *J* = 6.8 Hz, 1H), 7.79 (s, 1H), 7.67 - 7.55 (m, 2H), 7.48 (d, *J =* 9.1 Hz, 1H), 7.03 (s, 1H), 6.80 - 6.78 (m, 1H), 6.55 - 6.53 (m, 1H), 3.97 - 3.80 (m, 3H), 3.55- 3.31 (m, 4H), 2.97 - 2.79 (m, 3H), 2.31 (s, 6H). LCMS (ES, m/z): 457[M+H]⁺.

### Example 25: (2-(3-fluoropyridin-2-yl)indolizin-1-yl)((3aR,6aS)-5-(4-methylthienyl [3,2-d]pyrimidin-2-yl)hexahydropyrrolo[3,4-c]pyrrol-2(1H)-yl)methanone

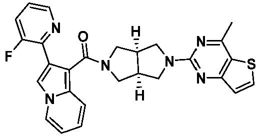

The starting material of the reaction, 2,4-dichlorofuro[3,2-d]pyrimidine, was replaced with 2,4-dichlorothieno[3,2-d]pyrimidine, and the method of Example 7 was followed to prepare the target compound. ¹H NMR (400 MHz, CDCl₃) δ 8.38 (d, *J* = 4.6 Hz, 1H), 7.90 (d, *J =* 7.0 Hz, 1H), 7.77 (d, *J =* 2.4 Hz, 1H), 7.72 (m, 1H), 7.44 (d, *J* = 9.1 Hz, 1H), 7.38 - 7.29 (m, 1H), 7.28 - 7.19 (m, 1H), 6.96 (s, 1H), 6.75 (m, 1H), 6.55 (t, *J* = 6.7 Hz, 1H), 3.94 - 3.81 (s, 3H), 3.69 - 3.49 (m, 4H), 3.09 - 2.91 (m, 3H), 2.61 (s, 3H). LCMS (ES, m/z): 499[M+H]⁺.

### Example 26: (2-(3-fluoropyridin-2-yl)indolizin-1-yl)((3aR,6aS)-5-(4-methyl-6,7-dihydro-5H-cy clopenta[d]pyrimidin-2-yl)hexahydropyrrolo[3,4-c]pyrrol-2(1H)-yl)methanone

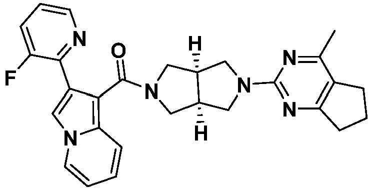

The starting material of the reaction, 2,4-dichlorofuro[3,2-d]pyrimidine, was replaced with 2,4-dichloro-6,7-dihydro-5H-cyclopenta[d]pyrimidine, and the method of Example 7 was followed to prepare the target compound. ¹H NMR (400 MHz, CDCl₃) δ 8.52 (d, *J =* 4.3 Hz, 1H), 7.97 (d, *J =* 6.4 Hz, 1H), 7.80 (d, *J =* 2.4 Hz, 1H), 7.53 (d, *J* = 8.9 Hz, 1H), 7.38 - 7. 36 (m, 1H), 7.07 - 7.10 (m, 1H), 6.81 - 6.78 (m, 1H), 6.56 (t, *J* = 6.7 Hz, 1H), 3.92-3.66 (m, 5H), 3.47 - 3.22 (m, 4H), 3.09 - 2.68 (m, 7H), 2.26 (s, 3H). LCMS (ES, m/z): 483[M+H]⁺,

### Example 27: ((3aR,6aS)-5-(3,6-dimethylpyrazin-2-yl)hexahydropyrrolo[3,4-c]pyrrol-2(1H)-yl) (2-(3-fluoropyridin-2-yl)indolizin-1-yl)methanone

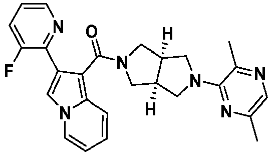

The starting material of the reaction, 2-chloro-4-methylfuro[3,2-d]pyrimidine, was replaced with 3-chloro-2,5-dimethylpyrazine, and the method of Example 7 was followed to prepare the target compound. ¹H NMR (400 MHz, CDCl₃) δ 8.54 (d, *J =* 4.8 Hz, 1H), 7.96 (d, *J* = 6.9 Hz, 1H), 7.84 (d, *J* = 2.4 Hz, 1H), 7.47 - 7.42 (m, 2H), 7.38 - 7.35 (m, 1H), 7.07 - 7.03(m, 1H), 6.79 - 6.67 (m, 1H), 6.52 (t, *J =* 6.7 Hz, 1H), 3.87 - 3.63 (m, 5H), 3.43 - 2.81 (m, 5H), 2.43 (s, 3H), 2.31 (s, 3H). LCMS (ES, m/z): 457[M+H]⁺.

### Example 28: (3aR,6aS)-5-(4,6-dimethylpyrimidin-2-yl)hexahydropyrrolo[3,4-c]pyrrol-2(1H)-y 1)(2-(thiophen-2-yl)indolizin-1-yl)methanone

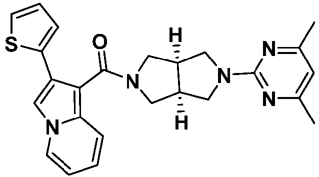

The starting material of the reaction, acetophenone, was replaced with 2-acetylthiophene, and the method of Example 1 was followed to prepare the target compound. ¹H NMR (400 MHz, CDCl₃) δ 7.87 (d, *J* = 6.9 Hz, 1H), 7.50 (d, *J* = 9.1 Hz, 1H), 7.39 (s, 1H), 7.14 (m, 2H), 6.95 (t, *J =* 4.4 Hz, 1H), 6.79 (m, 1H), 6.56 (t, *J =* 6.7 Hz, 1H), 6.29 (s, 1H), 3.91 (d, *J =* 48.6 Hz, 2H), 3.78 - 3.43 (m, 3H), 3.32 (s, 2H), 2.97 (s, 2H), 2.78 (s, 1H), 2.30 (s, 6H). LCMS (ES, m/z): 444[M+H]⁺.

### Example 29: ((3aR,6aS)-5-(4,6-dimethylpyrimidin-2-yl)hexahydropyrrolo[3,4-c]pyrrol-2(1H)-yl)(2-(pyrimidin-2-yl)pyrazolo[1,5-a]pyridin-3-yl)methanone

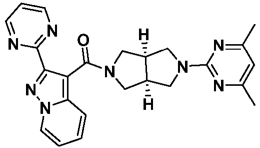

The starting material of the reaction, 2-iodothiophene, was replaced with 2-iodopyrimidine, and the method of Example 5 was followed to prepare the target compound. ¹H NMR (400 MHz, Chloroform-*d*): δ 8.74 (d, *J* = 4.7 Hz, 1H), 8.55 (d, *J* = 7.0 Hz, 2H), 8.04 (d, *J* = 7.9 Hz, 1H), 7.30 - 7.10 (m, 2H), 6.92 - 6.90 (m, 1H), 6.33 (s, 1H), 4.05 - 3.89 (m, 3H), 3.68 (dd, *J* = 11.7, 6.1 Hz, 2H), 3.52 - 3.39 (m, 2H), 3.18 - 2.90 (m, 3H), 2.32 (s, 6H). LCMS (ES, m/z): 441[M+H]⁺.

### Example 30: ((3aR,6aS)-5-(4,6-dimethylpyrimidin-2-yl)hexahydropyrrolo[3,4-c]pyrrol-2(1H)-yl) (6-fluoro-2-(pyridin-2-yl)pyrazolo[1,5-a]pyridin-3-yl)methanone

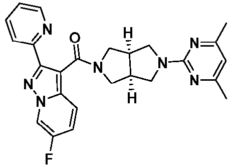

The starting material of the reaction, 1-aminopyridinium iodide, was replaced with 1-amino-3-fluoropyridinium iodide, and the method of Example 5 was followed to prepare the target compound. ¹H NMR (400 MHz, CDCl₃) δ 8.69 (d, *J* = 4.6 Hz, 1H), 8.37 (d, *J* = 6.9 Hz, 1H), 8.17 - 7.95 (m, 1H), 7.81 - 7.78 (m, 1H), 7.21 - 7.18 (m, 1H), 7.02 - 6.70 (m, 2H), 6.34 (s, 1H), 3.95 - 3.91 (m, 2H), 3.85 - 3.52 (m, 6H), 3.23 - 3.05 (m, 2H), 2.46 (s, 6H). LCMS (ES, m/z): 458[M+H]⁺.

### Example 31: ((3aR,6aS)-5-(4,6-dimethylpyrimidin-2-yl)hexahydropyrrolo[3,4-c]pyrrol-2(1H)-yl) (6-methyl-2-(pyridin-2-yl)pyrazolo[1,5-a]pyridin-3-yl)methanone

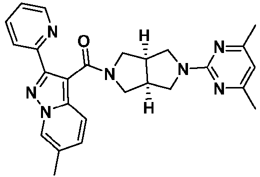

The starting material of the reaction, 1-aminopyridinium iodide, was replaced with 1-amino-3-methylpyridinium iodide, and the method of Example 5 was followed to prepare the target compound. ¹H NMR (400 MHz, CDCl₃) δ 8.61 (d, *J* = 4.9 Hz, 1H), 8.32 (d, *J* = 6.7 Hz, 1H), 8.11 - 7.89 (m, 1H), 7.68 - 7.63 (m, 1H), 7.16 - 7.14 (m, 1H), 7.06 - 6.78 (m, 2H), 6.31 (s, 1H), 3.91 - 3.88 (m, 2H), 3.81 - 3.41 (m, 6H), 3.21 - 3.01 (m, 2H), 2.41 (s, 6H). LCMS (ES, m/z): 458[M+H]⁺.

### Example 32: (5-(4,6-dimethoxypyrimidin-2-yl)hexahydropyrrolo[3,4-c]pyrrol-2(1H)-yl)(2-(3-fl uoropyridin-2-yl)indolizin-1-yl)methanone

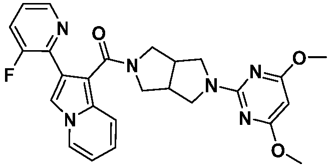

The starting material of the reaction, 2-chloro-4-methylfuro[3,2-d]pyrimidine, was replaced with 2-chloro-4,6-dimethoxypyrimidine, and the method of Example 7 was followed to prepare the target compound. ¹H NMR (400 MHz, CDCl₃) δ 8.48 (d, *J =* 4.4 Hz, 1H), 7.95 (d, *J* = 6.7 Hz, 1H), 7.81 (d, *J* = 2.4 Hz, 1H), 7.44 (d, *J* = 9.1 Hz, 1H), 7.40 - 7.78 (m, 1H), 7.11 - 7.08 (m, 1H), 6.77 (dd, *J =* 9.1, 6.5 Hz, 1H), 6.56 (t, *J =* 6.7 Hz, 1H), 6.06 (s, 1H), 4.06 - 3.73 (m, 9H), 3.62 - 3.41 (m, 5H), 3.07 - 2.83 (m, 3H). LCMS (ES, m/z): 489[M+H]⁺.

### Example 33: (5-(4,6-dimethylpyrimidin-2-yl)hexahydropyrrolo[3,4-c]pyrrol-2(1H)-yl)(2-pheny limidazo[1,2-b]pyridazin-3-yl)methanone

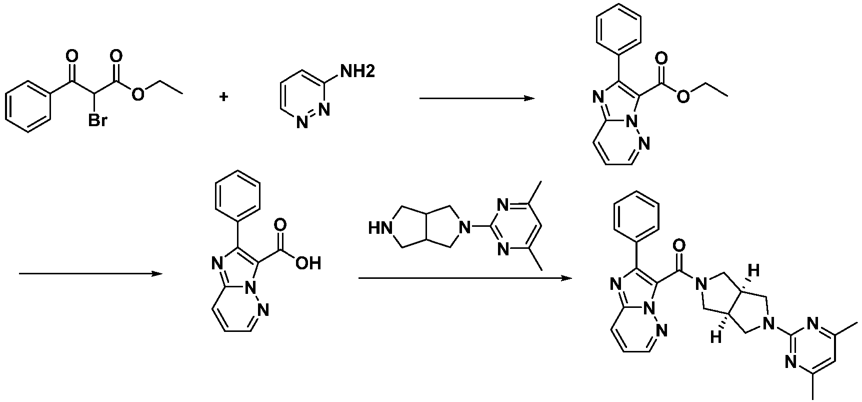

### 33.1. Preparation of ethyl 2-phenylimidazo[1,2-b]pyridazine-3-carboxylate

Ethyl 2-bromo-3-oxo-3-phenylpropionate (2 g, 7.38 mmol) and pyridazine-3-amine (0.74 g, 7.75 mmol) were dissolved in 10 mL of ethanol, and the solution was heated to 150 °C in a microwave reactor and left to react for 6 h. After the reaction was complete, the reaction mixture was cooled to room temperature, distilled under reduced pressure to remove the solvent, and subjected to column chromatography separation and purification (PE:EA = 10:1) to give 0.6 g of the product.

### 33.2. Preparation of 2-phenylimidazo[1,2-b]pyridazine-3-carboxylic acid

Ethyl 2-phenylimidazo[1,2-b]pyridazine-3-carboxylate (0.6 g, 2.24 mmol) and sodium hydroxide (0.18 g, 4.48 mmol) were added to 5 mL of water, and 2 mL of ethanol was added. The mixture was heated to 75 °C for reaction for 1 h. After the reaction was complete, the reaction mixture was cooled to room temperature and distilled under reduced pressure to remove ethanol, and the pH was adjusted to 1 with a 1 M HCl solution. The mixture was filtered, and the filter cake was dried to give 0.42 g, which was directly used in the next step without further purification.

### 33.3. Preparation of (5-(4,6-dimethylpyrimidin-2-yl)hexahydropyrrolo[3,4-c]pyrrol-2(1H)-yl)(2-pheny limidazo[1,2-b]pyridazin-3-yl)methanone

2-Phenylimidazo[1,2-b]pyridazine-3-carboxylic acid (0.4 g, 1.67 mmol), HATU (1.27 g, 3.34 mmol), Et₃N (0.3 g, 2.97 mmol), and acetonitrile (10 mL) were added to a 50 mL single-necked round-bottom flask under an ice bath and left to react for 10 min at that temperature. 2-(4,6-Dimethylpyrimidin-2-yl)octahydropyrrolo[3,4-c]pyrrole (0.4 g, 1.84 mmol) was added, and the mixture was left to react for 2 h at room temperature. After TLC analysis showed the completion of the reaction, 30 mL of water was added, and extraction was performed with 3 × 30 mL of DCM. The extract was then washed with 30 mL of saturated brine, dried over anhydrous sodium sulfate, filtered under vacuum, concentrated, and subjected to DCM/MeOH (20/1) silica gel column chromatography to give 0.3 g of the product. ¹H NMR (400 MHz, CDCl₃) δ 8.63 - 8.61 (m, 1H), 8.07 (dd, *J =* 9.1, 1.7 Hz, 1H), 7.88 - 7.74 (m, 2H), 7.52 - 7.41 (m, 2H), 7.28 - 7.23 (m, 1H), 6.44 (s, 1H), 3.95 - 3.91 (m, 2H), 3.85 - 3.52 (m, 6H), 3.23 - 3.05 (m, 2H), 2.46 (s, 6H). LCMS (ES, m/z): 458[M+H]⁺.

### Example 34: ((3aR,6aS)-5-(4,6-dimethylpyrimidin-2-yl)hexahydropyrrolo[3,4-c]pyrrol-2(1H)-yl)(2-(pyridin-4-yl)pyrazolo[1,5-a]pyridin-3-yl)methanone

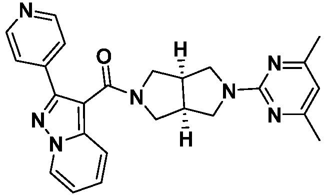

The starting material of the reaction, 2-iodothiazole, was replaced with 4-iodopyridine, and the method of Example 5 was followed to prepare the target compound. ¹H NMR (400 MHz, CDCl₃): δ 8.71 (d, *J* = 4.9 Hz, 1H), 8.23 - 8.14 (m, 2H), 7.74 - 7.61 (m, 2H), 7.32 - 7.11 (m, 2H), 6.90 (td, *J =* 6.9, 1.3 Hz, 1H), 6.33 (s, 1H), 3.95 - 3.81 (m, 3H), 3.62 - 3.35 (m, 4H), 3.18 - 2.95 (m, 3H), 2.31 (s, 6H). LCMS (ES, m/z): 440[M+H]⁺.

### Example 35: ((3aR,6aS)-5-(4,6-dimethylpyrimidin-2-yl)hexahydropyrrolo[3,4-c]pyrrol-2(1H)-yl)(3-methyl-2-(pyridin-2-yl)indolizin-1-yl)methanone

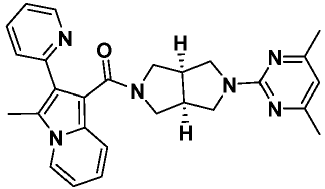

The starting material of the reaction, acetophenone, was replaced with 2-propionylpyridine, and the method of Example 1 was followed to prepare the target compound. ¹H NMR (400 MHz, CDCl₃) δ 8.56 (d, *J* = 4.9 Hz, 1H), 7.92 (d, *J* = 6.9 Hz, 1H), 7.67 - 7.55 (m, 2H), 7.48 (d, *J* = 9.1 Hz, 1H), 7.05 - 7.01 (m, 1H), 6.79 - 6.69 (m, 1H), 6.57 (t, *J =* 6.7 Hz, 1H), 6.29 (s, 1H), 3.95 - 3.76 (m, 3H), 3.53 - 3.28 (m, 4H), 2.93 - 2.73 (m, 3H), 2.48 (s, 3H), 2.30 (s, 6H). LCMS (ES, m/z): 453 [M+H]⁺.

### Example 36: [(3aR,6aS)-5-(4,6-dimethylpyrimidin-2-yl)hexahydropyrrolo[3,4-c]pyrrol-2(1H)-yl][2-(pyridin-3-yl)pyrazolo[1,5-a]pyridin-3-yl]methanone

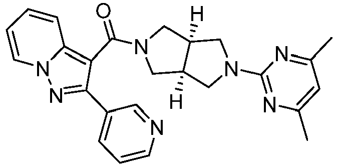

The starting material of the reaction, 2-iodothiophene, was replaced with 3-iodopyridine, and the method of Example 5 was followed to prepare the target compound. ¹H NMR (400 MHz, DMSO-*d*₆) δ 8.94 (s, 1H), 8.83 (d, *J* = 6.9 Hz, 1H), 8.52 (d, *J* = 4.7 Hz, 1H), 8.16 - 8.03 (m, 1H), 7.66 (d, *J* = 8.9 Hz, 1H), 7.48 - 7.30 (m, 2H), 7.07 (t, *J* = 7.0 Hz, 1H), 6.40 (s, 1H), 3.83 - 3.40 (m, 6H), 3.17 - 2.85 (m, 4H), 2.22 (s, 6H). LCMS (ES, m/z): 440[M+H]⁺.

### Example 37: [(3aR,6aS)-5-(4,6-dimethylpyrimidin-2-yl)hexahydropyrrolo[3,4-c]pyrrol-2(1H)-y l][6-fluoro-2-(thiophen-2-yl)pyrazolo[1,5-a]pyridin-3-yl]methanone

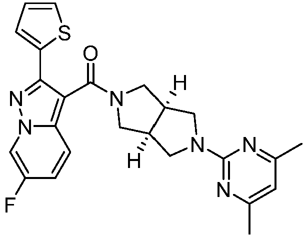

The starting material of the reaction, 1-aminopyridinium iodide, was replaced with 1-amino-3-fluoropyridinium iodide, and the method of Example 5 was followed to prepare the target compound. ¹H NMR (400 MHz, DMSO-d6) δ 8.46 - 8.39 (m, 2H), 7.54 - 7.26 (m, 2H), 7.22 - 7.07 (m, 1H), 6.93 - 6.82 (m, 1H), 6.85 - 6.61 (m, 1H), 6.41 (s, 1H), 4.01 - 3.85 (m, 2H), 3.75 - 3.56 (m, 2H), 3.53 -3.39 (m, 2H), 3.26 - 3.11 (m, 2H), 3.07 - 2.91 (m, 2H), 2.27 (s, 6H). LCMS (ES, m/z): 463[M+H]⁺.

### Pharmacological Test Examples:

### Test Example 1. In vitro Activity Test

(1) Preparation of a reaction buffer (1 × Stimulation buffer) required for the experiment: The 5 × Stimulation buffer and ddH₂O in the Cisbio IP-one kit were diluted in a ratio of 1:4 for later use.
(2) Compound preparation: The compound was diluted with DMSO to obtain a 5 mM stock solution, followed by a 3.16-fold dilution to obtain 10 gradients, and then the prepared compound was diluted with the Stimulation buffer to the corresponding concentrations (4 ×) for later use.
(3) Cell preparation: CHO-K1-OX1 and CHO-K1-OX2 cells on the culture dish were digested with trypsin, and the cells were washed off with culture medium and collected into 5 mL centrifuge tubes. The cells were centrifuged at 1000 rpm for 5 min, and the supernatant was discarded. 3 mL of PBS was added, and the mixture was gently pipetted to be mixed well. The cells were centrifuged again at 1000 rpm for 5 min, and the supernatant was discarded. The cells were resuspended in 1 × Stimulation buffer and counted using a Countstar cell counter, and the cell density was adjusted to 1.71 × 10⁶ cells/mL for later use.
(4) Cell addition: The cell suspensions were added to assay plates at 7 µL/well (i.e., about 12,000 cells/well).
(5) Compound addition: The compound diluted with the Stimulation buffer was added to the assay plates described above at 3.5 µL/well.
(6) Reaction and incubation: After gentle shaking, the assay plates were incubated at 37 °C for 30 min.
(7) EC₈₀ agonist addition: 4 × Orexin A (OX1 receptor) and 4 × Orexin 2 receptor agonist (OX2 receptor) solutions of EC₈₀ were added at 3.5 µL/well.
(8) Reaction and incubation: After gentle shaking, the assay plates were incubated at 37 °C for 45 min.
(9) Detection reagent addition: IP1-d2 and Anti-IP1 cryptate were separately diluted in a 1:20 ratio with the Lysis & detection buffer in the Cisbio IP-one detection kit, and the diluted IP1-d2 and Anti-IP1 cryptate described above were added to the assay plates, each at 3 µL/well. After shaking, the assay plates were left to stand at room temperature for 60 min.
(10) Experimental readings: The plate was read on Envision, readings from the 665 nm and 615 nm channels were taken, and the ratio of 665 nm/615 nm readings was calculated.

Based on the antagonistic effect values of the compound sample at different concentration test points, antagonistic curves of the compound sample against the orexin receptors were fitted using GraphPad Prism software, and the IC₅₀ was calculated. The results are shown in Table 1:

**Table 1**

| Compound | OX2 IC₅₀ (nM) | OX1 IC₅₀ (nM) |
|---|---|---|
| Seltorexant | +++ | + |
| Example 1 | ++ | - |
| Example 2 | ++ | - |
| Example 3 | +++ | - |
| Example 4 | +++ | - |
| Example 5 | +++ | - |
| Example 6 | +++ | - |
| Example 7 | +++ | - |
| Example 8 | +++ | - |
| Example 9 | ++ | - |
| Example 10 | ++ | - |
| Example 11 | ++ | - |
| Example 12 | ++ | - |
| Example 13 | ++ | - |
| Example 14 | ++ | - |
| Example 15 | +++ | - |
| Example 17 | +++ | - |
| Example 18 | +++ | - |
| Example 19 | +++ | - |
| Example 20 | +++ | - |
| Example 21 | +++ | - |
| Example 24 | +++ | - |
| Example 26 | +++ | - |
| Example 28 | +++ | - |
| Example 30 | +++ | - |
| Example 34 | +++ | - |
| Example 36 | +++ | - |
| Example 37 | +++ | - |

| | | |
|---|---|---|
| IC₅₀ value < 100 nM: +++; IC₅₀ value of 100-300 nM: ++; IC₅₀ value of 300-1000 nM: +; and IC₅₀ value > 1000 nM: - | | |

The data show that the compounds of the present invention had relatively good inhibitory activity against the OX2 receptor, and the inhibitory effects of the compounds on the OX2 receptor were significantly better than those on the OX1 receptor, indicating relatively good selectivity.

### Test Example 2. Determination of Rat Pharmacokinetic Parameters of Test Substances in Rat Plasma

Healthy male SD rats aged 6-9 weeks were selected and randomly divided into two groups, with 3 rats in each group. One group was given 1 mg/kg of the test compound by intravenous injection, and the other group was given 10 mg/kg of the test compound by gavage. Whole blood was collected from the animals in the intravenous group and the gavage group before administration and 0.083, 0.25, 0.5, 1.0, 2.0, 4.0, 7.0, 10.0, and 24.0 h after administration and centrifuged to obtain plasma samples.

All biological samples were quantitatively analyzed using the LC-MS/MS method. Pharmacokinetic parameters were calculated using the non-compartmental model linear-log trapezoidal method using WinNonlinTMVersion7.0 (Pharsight, Mountain View, CA) pharmacokinetic software. AUC₀₋ₗₐₛₜ represents the area under the plasma concentration-time curve from time point zero to the last detectable concentration time point; PO represents oral administration; iv represents intravenous, Cₘₐₓ represents the peak concentration, and F% represents oral bioavailability. The results are shown in Table 2:

**Table 2**

| PK parameters | | Seltorexant | Example 3 | Example 30 | Example 34 |
|---|---|---|---|---|---|
| iv 1mg/kg | Cₘₐₓ(ng/mL) | 1101 | 875.3 | 439.1 | 715.1 |
| | AUC₀₋ₗₐₛₜ(h*ng/mL) | 442.4 | 355.4 | 143.1 | 221 |
| PO 10mg/kg | Cₘₐₓ(ng/mL) | 942.7 | 2775 | 668.1 | 925 |
| | AUC₀₋ₗₐₛₜ(h*ng/mL) | 701.4 | 2002.2 | 353.1 | 560 |
| | F (%) | 15.9 | 56.3 | 24.6 | 25.3 |

The data show that in the rat pharmacokinetic evaluation experiment, the example compounds of the present invention exhibited relatively good in vivo exposure and relatively good bioavailability after administration.

### Test Example 3. Autonomous Activities of Mice

Male ICR mice aged 6-9 weeks were randomly divided into groups based on body weight balance, with 8 or 9 mice per group, and the groups were given blank vehicle and 10, 30, and 100 mg/kg of the test compound, respectively. The animals were placed in a test chamber immediately after administration, and the activity distances of the animals within 60 min were recorded and analyzed with Top Scan Version 3.0. The total activity distances of the animals in each administration group of the test samples and the blank vehicle group were compared to determine whether the test samples had a significant effect on the autonomous activities of the animals. Experimental data were expressed as mean ± standard deviation (Mean ± SD). One-way ANOVA analysis was performed using SPSS 21.0 statistical software, and pairwise comparisons were performed using Dunnett's test, with p < 0.05 represented by *.

**Table 3**

| Group | Dose (mg/kg) | Movement distance (m) | Inhibition rate (%) |
|---|---|---|---|
| Vehicle (20-%β-cyclodextrin) | - | 65.18±13.29 | - |
| Seltorexant | 10 | 62.39±23.31 | 4.27 |
| | 30 | 36.95±9.75* | 43.30 |
| | 100 | 32.68±13.49* | 49.86 |
| Example 3 | 10 | 68.58±24.42 | -5.22 |
| | 30 | 58.78±18.31 | 9.82 |
| | 100 | 38.69±13.98* | 40.63 |
| Example 36 | 10 | 44.27±13.76* | 32.08 |
| | 30 | 42.78±20.27* | 34.37 |
| | 100 | 27.81±19.12* | 57.33 |
| Example 34 | 10 | 59.84±19.89 | 8.19 |
| | 30 | 46.84±16.61* | 28.13 |
| | 100 | 32.36±20.35* | 50.36 |
| Example 37 | 10 | 61.90±16.82 | 5.03 |
| | 30 | 40.89±21.03* | 37.27 |
| | 100 | 39.42±13.92* | 39.52 |
| Example 5 | 10 | 63.64±33.70 | 2.37 |
| | 30 | 34.56±10.71* | 46.97 |
| | 100 | 33.13±14.64* | 49.17 |

Conclusion: The compounds of the present invention can significantly reduce the spontaneous activity distance of mice.

### Test Example 4. Pentobarbital Sodium Synergistic Sleep Test

Male ICR mice aged 6-9 weeks were randomly divided into groups based on body weight balance, with 8 mice per group, and the groups were given blank vehicle and 3, 10, and 30 mg/kg of the test compound, respectively. After 5 min, pentobarbital sodium was intraperitoneally injected at 45 mg/kg. The moments when righting reflexes disappeared and the moments when righting reflexes reappeared in mice were recorded. Sleep latency = moment when righting reflex disappeared - moment when pentobarbital sodium was administered, and sleep duration = moment when righting reflex reappeared - moment when righting reflex disappeared. The sleep latency and sleep duration of each administration group of the test samples and the blank vehicle group were compared to determine whether the test samples had a significant effect on the sleep latency and sleep duration of the animals. Experimental data were expressed as mean ± standard deviation (Mean ± SD). One-way ANOVA analysis was performed using SPSS 21.0 statistical software, and pairwise comparisons were performed using Dunnett's test, with p < 0.05 represented by *.

**Table 4**

| Compound | Dose (mg/kg) | Number of animals | Sleep latency (min) | Sleep duration (min) |
|---|---|---|---|---|
| Vehicle (20-%P-cyclodextrin) | - | 8 | 16.74±11.49 | 30.86±21.98 |
| Seltorexant | 30 | 8 | 5.05±2.67* | 60.41±11.91* |
| Example 30 | 3 | 8 | 5.03±0.51* | 62.34±17.87* |
| | 10 | 8 | 5.17±0.81* | 66.79±14.88* |
| | 30 | 8 | 6.52±5.25* | 65.61±19.83* |
| Example 36 | 3 | 8 | 10.21±4.49* | 50.35±9.35* |
| | 10 | 8 | 10.58±5.65 | 38.89±10.66 |
| | 30 | 8 | 8.84±3.67* | 56.19±27.74* |
| Example 3 | 3 | 8 | 15.21±4.60 | 57.88±23.72 |
| | 10 | 8 | 17.72±6.04 | 62.73±29.84* |
| | 30 | 8 | 24.28±8.97 | 63.15±34.38* |

Conclusion: The compounds of the present invention can shorten the sleep latency of mice and prolong the sleep duration of mice.

## Claims

1. A compound represented by formula I or a pharmaceutically acceptable salt thereof, a stereoisomer thereof, or a tautomer thereof, 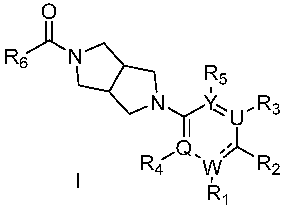
wherein R₁, R₂, R₃, R₄, and R₅ are absent or independently selected from H, halogen, C₁-C₈ linear or branched alkyl, and C₁-C₈ alkoxy;
or R₁ and R₂ form C₃-C₈ cycloalkyl, aryl, heteroaryl, or a 3-8 membered heterocyclic ring; preferably, R₁ and R₂ form C₃-C₈ cycloalkyl, 6-10 membered monocyclic or bicyclic aryl, 5-10 membered monocyclic or bicyclic heteroaryl containing 1-5 heteroatoms independently selected from nitrogen, oxygen, or sulfur, or a 3-8 membered monocyclic or bicyclic heterocyclic ring containing 1-3 heteroatoms independently selected from nitrogen, oxygen, or sulfur;
Q, W, Y, and U are independently selected from C and N, and Y and Q are not simultaneously C;
--- is a single bond or a double bond;
R₆ is selected from formula II, formula III, and formula IV: 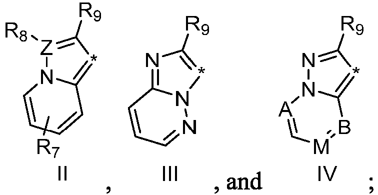
R₉ is optionally substituted heteroaryl, an optionally substituted aromatic ring, an optionally substituted 3-8 membered heterocyclic ring, or optionally substituted C₃-C₈ cycloalkyl, and a substituent is selected from halogen, C₁-C₈ linear or branched alkyl, C₁-C₈ alkoxy, and haloalkyl; preferably, R₉ is optionally substituted 5-10 membered monocyclic or bicyclic heteroaryl containing 1-5 heteroatoms independently selected from nitrogen, oxygen, or sulfur, an optionally substituted 6-10 membered monocyclic or bicyclic aromatic ring group, an optionally substituted 3-8 membered monocyclic or bicyclic heterocyclic ring containing 1-3 heteroatoms independently selected from nitrogen, oxygen, or sulfur, or optionally substituted C₃-C₈ cycloalkyl; in formula II, Z is selected from C and N;
R₇ is selected from H, halogen, and C₁-C₈ linear or branched alkyl;
R₈ is absent or independently selected from H, halogen, and C₁-C₈ linear or branched alkyl;
in formula IV, A, B, and M are independently selected from CH and N, and A, B, and M are not simultaneously CH.

2. The compound represented by formula I or the pharmaceutically acceptable salt thereof, the stereoisomer thereof, or the tautomer thereof according to claim 1, wherein:
the C₁-C₈ linear or branched alkyl is selected from C₁-C₅ linear or branched alkyl; and/or
the C₁-C₈ alkoxy is selected from C₁-C₅ alkoxy; and/or
the C₃-C₈ cycloalkyl is selected from C₃-C₆ cycloalkyl; and/or
the 3-8 membered heterocyclic ring comprises 1-3 heteroatoms, and the heteroatoms are selected from O, N, and S.

3. The compound represented by formula I or the pharmaceutically acceptable salt thereof, the stereoisomer thereof, or the tautomer thereof according to claim 2, wherein:
the halogen is fluorine, chlorine, bromine, or iodine; and/or
the C₁-C₅ linear or branched alkyl is selected from methyl, ethyl, propyl, isopropyl, butyl, isobutyl, and pentyl; and/or
the C₁-C₅ alkoxy is selected from methoxy, ethoxy, propoxy, butoxy, and pentyloxy; and/or
the C₃-C₆ cycloalkyl is selected from cyclopropyl, cyclobutyl, cyclopentyl, cyclopentenyl, and cyclohexyl; and/or
the heteroaryl is selected from pyridyl, pyridazinyl, triazinyl, pyrimidinyl, thienyl, furanyl, oxazolyl, thiazolyl, thiadiazolyl, oxadiazolyl, isoxazolyl, pyrazolyl, imidazolyl, pyrrolyl, pyranyl, pyrazinyl, and triazolyl; and/or
the haloalkyl is selected from fluoromethyl, difluoromethyl, trifluoromethyl, fluoroethyl, difluoroethyl, and trifluoroethyl.

4. The compound represented by formula I or the pharmaceutically acceptable salt thereof, the stereoisomer thereof, or the tautomer thereof according to any one of claims 1-3, wherein: the compound of formula I is represented by formula V: 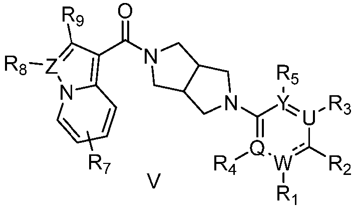
wherein R₁, R₂, R₃, R₄, and R₅ are absent or independently selected from H, fluorine, chlorine, methyl, ethyl, propyl, isopropyl, methoxy, ethoxy, and propoxy;
or R₁ and R₂ form C₃-C₆ cycloalkyl, 6-10 membered monocyclic or bicyclic aryl, 5-10 membered monocyclic or bicyclic heteroaryl containing 1-5 heteroatoms independently selected from nitrogen, oxygen, or sulfur, or a 3-8 membered monocyclic or bicyclic heterocyclic ring containing 1-3 heteroatoms independently selected from nitrogen, oxygen, or sulfur;
Q, W, Y, and U are independently selected from C and N, and Y and Q are not simultaneously C;
--- is a single bond or a double bond;
Z is selected from C and N;
R₇ is selected from H, fluorine, chlorine, methyl, ethyl, propyl, and isopropyl;
R₈ is absent or independently selected from H, fluorine, chlorine, methyl, ethyl, propyl, and isopropyl;
R₉ is optionally substituted 5-10 membered monocyclic or bicyclic heteroaryl containing 1-5 heteroatoms independently selected from nitrogen, oxygen, or sulfur, an optionally substituted 6-10 membered monocyclic or bicyclic aromatic ring group, an optionally substituted 3-8 membered monocyclic or bicyclic heterocyclic ring containing 1-3 heteroatoms independently selected from nitrogen, oxygen, or sulfur,
or optionally substituted C₃-C₈ cycloalkyl, and a substituent is selected from fluorine, chlorine, methyl, ethyl, propyl, isopropyl, methoxy, ethoxy, propoxy, trifluoromethyl, fluoromethyl, and difluoromethyl.

5. The compound represented by formula I or the pharmaceutically acceptable salt thereof, the stereoisomer thereof, or the tautomer thereof according to claim 4, wherein: the compound of formula V is represented by formula V-1: 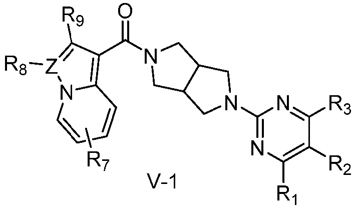
wherein R₁, R₂, and R₃ are independently selected from H, fluorine, chlorine, methyl, ethyl, propyl, isopropyl, methoxy, ethoxy, and propoxy;
or R₁ and R₂ form C₃-C₆ cycloalkyl, 6-10 membered monocyclic or bicyclic aryl, 5-10 membered monocyclic or bicyclic heteroaryl containing 1-5 heteroatoms independently selected from nitrogen, oxygen, or sulfur, or a 3-8 membered monocyclic or bicyclic heterocyclic ring containing 1-3 heteroatoms independently selected from nitrogen, oxygen, or sulfur;
Z is selected from C and N;
R₇ is selected from H, fluorine, chlorine, methyl, ethyl, propyl, and isopropyl;
Rg is absent or independently selected from H, fluorine, chlorine, methyl, ethyl, propyl, and isopropyl;
R₉ is optionally substituted 5-10 membered monocyclic or bicyclic heteroaryl containing 1-5 heteroatoms independently selected from nitrogen, oxygen, or sulfur, an optionally substituted 6-10 membered monocyclic or bicyclic aromatic ring group, an optionally substituted 3-8 membered monocyclic or bicyclic heterocyclic ring containing 1-3 heteroatoms independently selected from nitrogen, oxygen, or sulfur, or optionally substituted C₃-C₈ cycloalkyl; the substituent is selected from fluorine, chlorine, methyl, ethyl, propyl, isopropyl, methoxy, ethoxy, propoxy, trifluoromethyl, fluoromethyl, and difluoromethyl.

6. The compound represented by formula I or the pharmaceutically acceptable salt thereof, the stereoisomer thereof, or the tautomer thereof according to any one of claims 1-3, wherein: the compound represented by formula I is represented by formula VI: 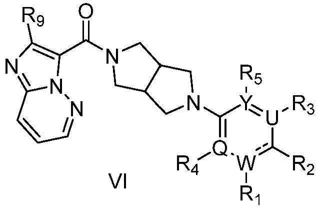
wherein R₁, R₂, R₃, R₄, and R₅ are absent or independently selected from H, fluorine, chlorine, methyl, ethyl, propyl, isopropyl, methoxy, ethoxy, and propoxy;
or R₁ and R₂ form C₃-C₆ cycloalkyl, 6-10 membered monocyclic or bicyclic aryl, 5-10 membered monocyclic or bicyclic heteroaryl containing 1-5 heteroatoms independently selected from nitrogen, oxygen, or sulfur, or a 3-8 membered monocyclic or bicyclic heterocyclic ring containing 1-3 heteroatoms independently selected from nitrogen, oxygen, or sulfur;
Q, W, Y, and U are independently selected from C and N, and Y and Q are not simultaneously C;
--- is a single bond or a double bond;
R₉ is optionally substituted 5-10 membered monocyclic or bicyclic heteroaryl containing 1-5 heteroatoms independently selected from nitrogen, oxygen, or sulfur, an optionally substituted 6-10 membered monocyclic or bicyclic aromatic ring group, an optionally substituted 3-8 membered monocyclic or bicyclic heterocyclic ring containing 1-3 heteroatoms independently selected from nitrogen, oxygen, or sulfur, or optionally substituted C₃-C₈ cycloalkyl; the substituent is selected from fluorine, chlorine, methyl, ethyl, propyl, isopropyl, methoxy, ethoxy, propoxy, trifluoromethyl, fluoromethyl, and difluoromethyl.

7. The compound represented by formula I or the pharmaceutically acceptable salt thereof, the stereoisomer thereof, or the tautomer thereof according to any one of claims 1-3, wherein: the compound represented by formula I is represented by formula VII: 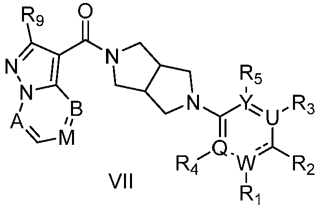
wherein R₁, R₂, R₃, R₄, and R₅ are absent or independently selected from H, fluorine, chlorine, methyl, ethyl, propyl, isopropyl, methoxy, ethoxy, and propoxy;
or R₁ and R₂ form C₃-C₆ cycloalkyl, 6-10 membered monocyclic or bicyclic aryl, 5-10 membered monocyclic or bicyclic heteroaryl containing 1-5 heteroatoms independently selected from nitrogen, oxygen, or sulfur, or a 3-8 membered monocyclic or bicyclic heterocyclic ring containing 1-3 heteroatoms independently selected from nitrogen, oxygen, or sulfur;
Q, W, Y, and U are independently selected from C and N, and Y and Q are not simultaneously C;
--- is a single bond or a double bond;
A, B, and M are selected from CH and N, and A, B, and M are not simultaneously CH;
R₉ is optionally substituted 5-10 membered monocyclic or bicyclic heteroaryl containing 1-5 heteroatoms independently selected from nitrogen, oxygen, or sulfur, an optionally substituted 6-10 membered monocyclic or bicyclic aromatic ring group, an optionally substituted 3-8 membered monocyclic or bicyclic heterocyclic ring containing 1-3 heteroatoms independently selected from nitrogen, oxygen, or sulfur, or optionally substituted C₃-C₈ cycloalkyl; the substituent is selected from fluorine, chlorine, methyl, ethyl, propyl, isopropyl, methoxy, ethoxy, propoxy, trifluoromethyl, fluoromethyl, and difluoromethyl.

8. The compound represented by formula I or the pharmaceutically acceptable salt thereof, the stereoisomer thereof, or the tautomer thereof according to any one of claims 1-7, wherein: the compound of formula I is selected from any one of the following compounds: 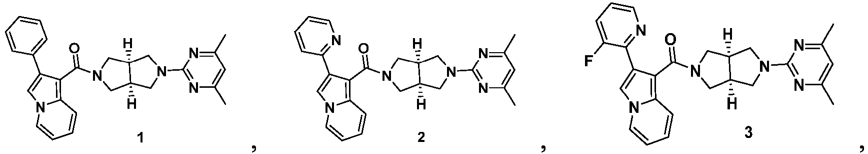 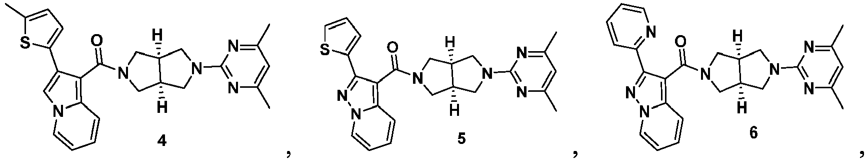 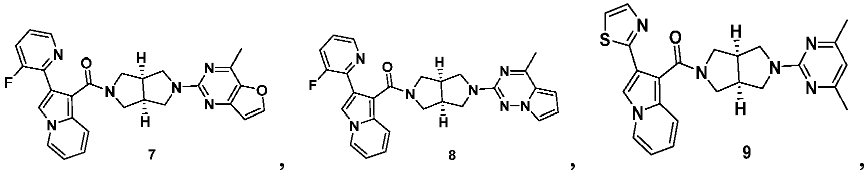 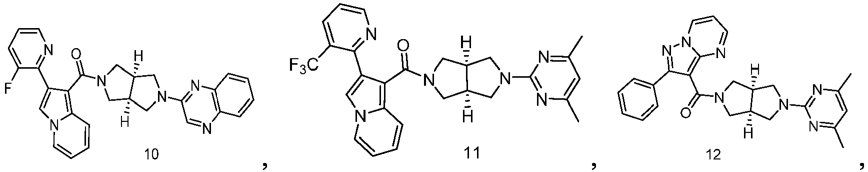 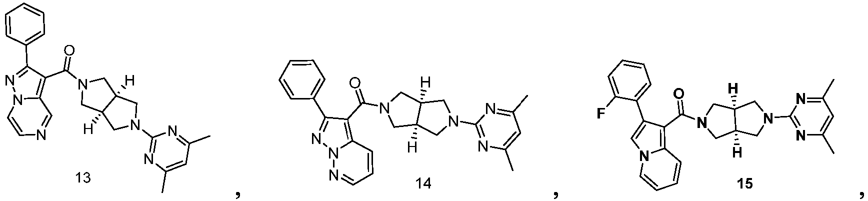 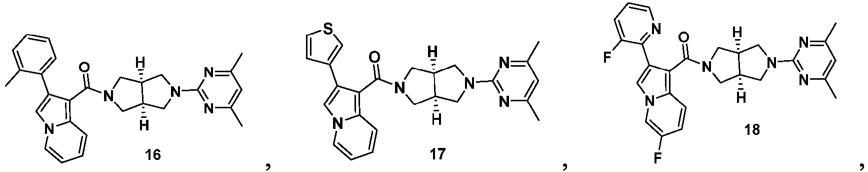 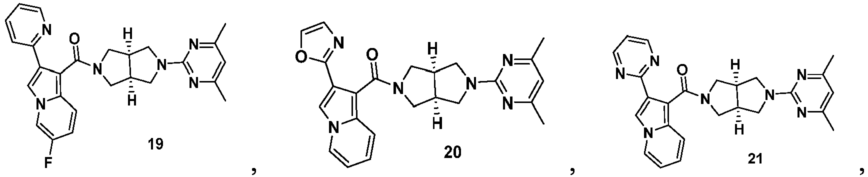 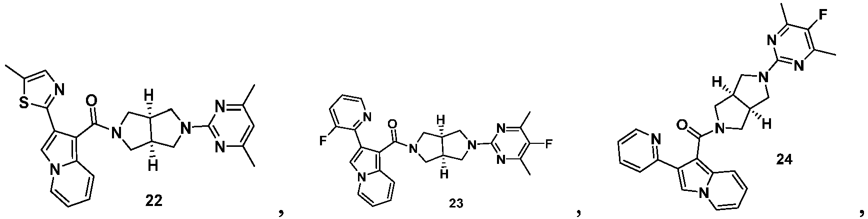 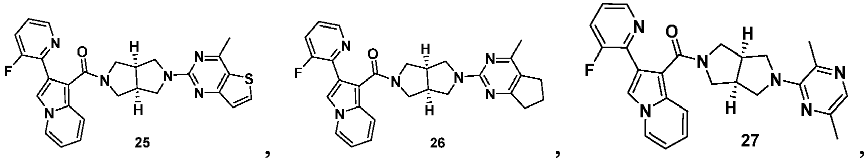 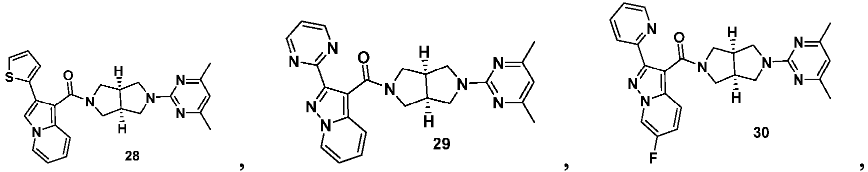 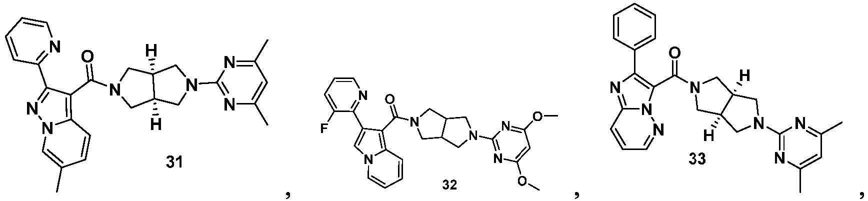 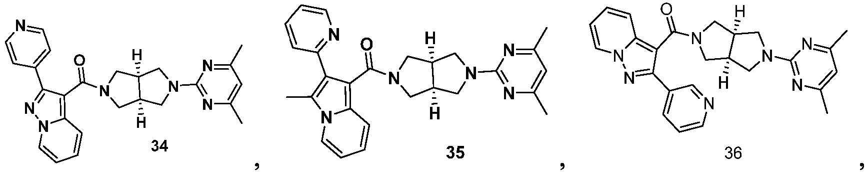 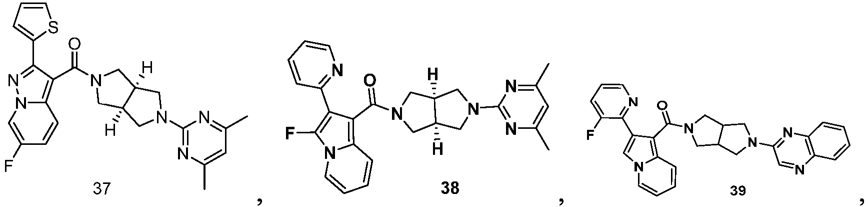 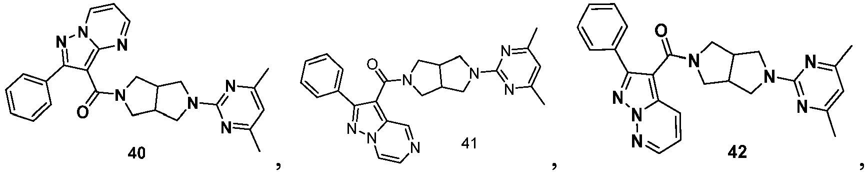 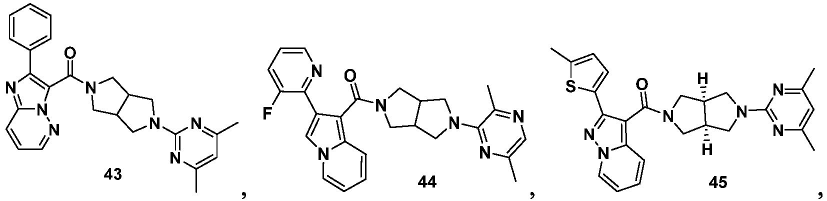 and

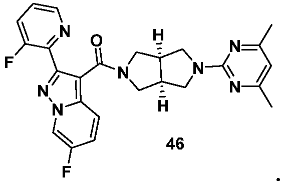

9. A pharmaceutical composition, comprising the compound of formula I or the pharmaceutically acceptable salt thereof, the stereoisomer thereof, or the tautomer thereof according to any one of claims 1-8, and optionally further comprising a pharmaceutically acceptable carrier or a combination thereof.

10. Use of the compound or the pharmaceutically acceptable salt thereof, the stereoisomer thereof, or the tautomer thereof according to any one of claims 1-8, or the pharmaceutical composition according to claim 9 in the preparation of a medicament, wherein the medicament is used for treating an orexin receptor-related disease.

11. The use according to claim 10, wherein the orexin receptor-related disease is sleep disorder, depression, anxiety disorder, panic disorder, obsessive-compulsive disorder, affective neuropathy, depressive neuropathy, anxiety neuropathy, mood disorder, panic attack disorder, behavioral disorder, emotional disturbance, post-traumatic stress disorder, psychosis, schizophrenia, manic depression, delirium, dementia, drug dependence, addiction, cognitive disorder, Alzheimer's disease, Parkinson's disease, dyskinesia, eating disorder, headache, migraine, or pain.

12. The use according to claim 10, wherein the orexin receptor-related disease is sleep disorder.
